# EUROPÄISCHE PATENTSCHRIFT

(11) **EP 3 370 653 B1**
(45) Veröffentlichungstag und Bekanntmachung des Hinweises auf die Patenterteilung: **05.02.2020**
(21) Anmeldenummer: 16791397.9
(22) Anmeldetag: 07.11.2016
(51) Int. Cl.: A61F 2/38

(54) **KNIEGELENKENDOPROTHESE**
KNEE-JOINT ENDOPROSTHESIS
ENDOPROTHÈSE DE L'ARTICULATION DU GENOU

(30) Priorität: 06.11.2015 DE 102015119105
(43) Veröffentlichungstag der Anmeldung: 12.09.2018
(73) Patentinhaber: Aesculap AG, 78532 Tuttlingen (DE)
(72) Erfinder: BÖTTIGER, Roland, 78604 Rietheim-Weilheim (DE)
(74) Vertreter: Hoeger, Stellrecht & Partner Patentanwälte mbB
(86) Internationale Anmeldenummer: PCT/EP2016/076768
(87) Internationale Veröffentlichungsnummer: WO 2017/077098

(56) Entgegenhaltungen:
- EP-A1- 2 042 133
- WO-A1-2015/165955
- DE-A1- 2 522 377
- US-A1- 2009 299 482

## Beschreibung

Die vorliegende Erfindung betrifft eine Kniegelenkendoprothese mit einer Tibiakomponente und einer Femurkomponente und einem Scharniergelenk zum drehgelenkigen Koppeln der Tibiakomponente und der Femurkomponente um eine Drehachse, welches Scharniergelenk ein erstes Gelenkelement und ein mit diesem um die Drehachse verdrehbar gekoppeltes zweites Gelenkelement umfasst, wobei eine Verbindungsvorrichtung mit mindestens einem ersten Verbindungselement und mindestens einem zweiten Verbindungselement zum Verbinden des ersten Gelenkelements mit der Femurkomponente vorgesehen ist, welche Verbindungseinrichtung eine Verbindungsstellung, in welcher das mindestens eine erste Verbindungselement oder das mindestens eine zweite Verbindungselement kraft- und/oder formschlüssig in Eingriff stehen, und eine Montagestellung, in welcher das erste Gelenkelement und die Femurkomponente vollständig voneinander getrennt sind, definiert, wobei das mindestens eine erste Verbindungselement dem ersten Gelenkelement zugeordnet oder an diesem angeordnet oder ausgebildet ist und wobei das mindestens eine zweite Verbindungselement der Femurkomponente zugeordnet oder an dieser angeordnet oder ausgebildet ist, wobei die Verbindungseinrichtung eine Verbindungsrichtung definiert, in welcher das mindestens eine erste Verbindungselement und das mindestens eine zweite Verbindungselement relativ zueinander bewegbar sind zum Überführen der Verbindungseinrichtung von der Montagestellung in die Verbindungsstellung, wobei die Verbindungsrichtung quer zur Drehachse verläuft. Insbesondere kann sie senkrecht zur Drehachse verlaufen.

Eine Kniegelenkendoprothese mit einer Tibiakomponente und einer Femurkomponente und einem Scharniergelenk zum drehgelenkigen Koppeln der Tibiakomponente und der Femurkomponente um eine Drehachse, welches Scharniergelenk ein erstes Gelenkelement und ein mit diesem um die Drehachse verdrehbar gekoppeltes zweites Gelenkelement umfasst, wobei eine Verbindungsvorrichtung mit mindestens einem ersten Verbindungselement und mindestens einem zweiten Verbindungselement zum Verbinden des ersten Gelenkelements mit der Femurkomponente vorgesehen ist, welche Verbindungseinrichtung eine Verbindungsstellung, in welcher das mindestens eine erste Verbindungselement oder das mindestens eine zweite Verbindungselement kraft- und/oder formschlüssig in Eingriff stehen, und eine Montagestellung, in welcher das erste Gelenkelement und die Femurkomponente vollständig voneinander getrennt sind, definiert, wobei das mindestens eine erste Verbindungselement dem ersten Gelenkelement zugeordnet oder an diesem angeordnet oder ausgebildet ist und wobei das mindestens eine zweite Verbindungselement der Femurkomponente zugeordnet oder an dieser angeordnet oder ausgebildet ist, wobei die Verbindungseinrichtung eine Verbindungsrichtung definiert, in welcher das mindestens eine erste Verbindungselement und das mindestens eine zweite Verbindungselement relativ zueinander bewegbar sind zum Überführen der Verbindungseinrichtung von der Montagestellung in die Verbindungsstellung, ist in verschiedenen Ausführungsformen bekannt. Beispielsweise ist eine solche in der EP 2 272 468 B1 beschrieben. Bei dieser Kniegelenkendoprothese werden die Femurkomponente und die Tibiakomponente unabhängig voneinander implantiert, wobei vor der Implantation das Scharniergelenk an der Femurkomponente ausgebildet wird. Eine Kopplung der Femurkomponente und der Tibiakomponente findet dann während der Operation statt. Dabei wird eine Gelenkzapfenverlängerung mit einem Gelenkzapfen durch zusammenwirkende Verriegelungskonen gekoppelt.

Ein Nachteil der bekannten Kniegelenkendoprothese ist insbesondere, dass zum Koppeln der Femurkomponente und der Tibiakomponente die Gelenkzapfenverlängerung in die am Gelenkzapfen vorgesehene Gelenkzapfenaufnahme eingeschlagen werden muss, um eine sichere Verbindung gewährleisten zu können. Bei diesem Einschlagen wird auch ein Impuls auf die bereits implantierte Tibiakomponente übertragen, sodass die Gefahr besteht, dass sich diese in unbeabsichtigter Weise lockern kann.

Gekoppelte orthopädische Prothesen sin in der EP 2 042 133 A1 offenbart. Aus der WO 2015/165955 A1 und der DE 25 22 377 A1 sind Kniegelenkendoprothesen bekannt. In der US 2009/0299482 A1 sind Kniegelenkprothesensysteme beschrieben.

Es ist daher eine Aufgabe der vorliegenden Erfindung, eine Kniegelenkendoprothese der eingangs beschriebenen Art so zu verbessern, dass sie möglichst einfach implantiert werden kann.

Diese Aufgabe wird bei einer Kniegelenkendoprothese der eingangs beschriebenen Art erfindungsgemäß dadurch gelöst, dass das erste Gelenkelement in Form einer Scharnierachse ausgebildet ist und dass das zweite Gelenkelement eine Scharnierachsenaufnahme aufweist, die von der Scharnierachse durchsetzt ist.

Die erfindungsgemäß vorgeschlagene Weiterbildung ermöglicht es insbesondere, die Femurkomponente und die Tibiakomponente insbesondere intraoperativ zu koppeln durch Überführen der Verbindungseinrichtung von der Montagestellung in die Verbindungsstellung. Dazu wird, anders als beispielsweise bei der aus der EP 2 272 468 B1 bekannten Kniegelenkendoprothese, das erste Gelenkelement quer zur Drehachse mit der Femurkomponente in Eingriff gebracht und nicht parallel zu einer vom ersten Gelenkelement definierten Längsachse, die auch die Drehachse definiert. Durch diese Form der Kopplung der Femurkomponente mit der Tibiakomponente, indem also das erste Gelenkelement mit der Femurkomponente gekoppelt wird zum Verbinden der unabhängig voneinander implantierten Femurkomponente mit der Tibiakomponente, kann ein Kraftstoß auf die Tibiakomponente vermieden werden. Die insbesondere für eine Kopplung zwischen dem zweiten Gelenkelement und der Tibiakomponente relevante Verbindung kann dann von einem Operateur bereits außerhalb des Körpers des Patienten vorgenommen werden. Eine Kopplung der Femurkomponente und der Tibiakomponente kann dann durch eine eingeschränkte Bewegung der beiden Komponenten relativ zueinander erreicht werden, nämlich indem die ersten und zweiten Verbindungselemente durch eine Bewegung in der Verbindungsrichtung quer zur Drehachse, insbesondere senkrecht zur Drehachse, miteinander in Eingriff gebracht werden. Zudem ermöglicht es die vorgeschlagene Weiterbildung, vollständig auf Verriegelungskonen zu verzichten, sodass die Gefahr eines Lösens einer solchen Verriegelungskonusverbindung nach der Implantation ausgeschlossen werden kann. Gemäß der Erfindung ist vorgesehen, dass das erste Gelenkelement in Form einer Scharnierachse ausgebildet ist und dass das zweite Gelenkelement eine Scharnierachsenaufnahme aufweist, die von der Scharnierachse durchsetzt ist. Einer Scharnierachse im Sinne dieser Patentanmeldung ist eine mechanische Achse, die Teil des Scharniergelenks ist. Sie definiert insbesondere die Drehachse, also die mathematische Achse, um die sich das erste und das zweite Gelenkelement relativ zueinander verdrehen können.

Vorzugsweise ist das zweite Gelenkelement an der Tibiakomponente gehalten oder mit dieser koppelbar. Damit lässt sich das Scharniergelenk vor der Implantation der Tibiakomponente bereits an der Tibiakomponente anordnen, also gerade anders als bei der aus der EP 2 272 468 B1 bekannten Kniegelenkendoprothese.

Günstig ist es, wenn die Verbindungseinrichtung derart ausgebildet ist, dass sie nach einer unabhängigen Implantation der Femurkomponente und der Tibiakomponente voneinander von der Montagestellung in die Verbindungsstellung überführbar ist. Wie bereits oben beschrieben hat dies den Vorteil, dass die Femurkomponente und die Tibiakomponente unabhängig voneinander implantiert werden können. Zudem kann durch die vorgeschlagene Weiterbildung verhindert werden, dass zum Koppeln der Femurkomponente und der Tibiakomponente ein Kraftstoß in Richtung auf die Tibiakomponente hin ausgeübt werden muss.

Besonders einfach und kompakt ausbilden lässt sich die Kniegelenkendoprothese, wenn eine vom ersten Gelenkelement definierte Längsachse die Drehachse definiert. Beispielsweise kann das erste Gelenkelement einen zylindrischen Lagerbolzen umfassen, der im mechanischen Sinne eine Scharnierachse des Scharniergelenks bildet und der mit seiner Längsachse die Drehachse definiert.

Vorteilhaft ist es, wenn das mindestens eine erste Verbindungselement in Form eines Verbindungsvorsprungs und wenn das mindestens eine zweite Verbindungselement in Form einer Verbindungsaufnahme ausgebildet ist oder umgekehrt. Derart ausgebildete Verbindungselemente lassen sich auf einfache Weise in Eingriff und gegebenenfalls auch wieder außer Eingriff bringen.

Vorzugsweise erstreckt sich die Verbindungsaufnahme parallel zur Verbindungsrichtung und weist eine Einfuhröffnung zum Einführen des Verbindungsvorsprungs parallel zur Verbindungsrichtung auf. Eine derart angeordnete und ausgebildete Verbindungsaufnahme ermöglicht es insbesondere, das erste Gelenkelement quer zur Drehachse mit dem mindestens einen ersten Verbindungselement in die Verbindungsaufnahme einzuführen.

Um eine sichere Verbindung zwischen dem ersten Gelenkelement und der Femurkomponente sicherstellen zu können, ist es günstig, wenn die Kniegelenkendoprothese zwei erste und/oder zwei zweite Verbindungselemente aufweist.

Besonders einfach und kompakt ausbilden lässt sich das Scharniergelenk, wenn die Scharnierachsenaufnahme in Form einer Durchgangsbohrung ausgebildet ist.

Vorzugsweise bilden die Scharnierachse und die Scharnierachsenaufnahme ein Gleitlager. So kann eine Bewegung des ersten und des zweiten Gelenkelements relative zueinander mit minimalem Spiel vorgegeben werden.

Ferner kann es vorteilhaft sein, wenn die Scharnierachse einen Scharnierachsenkern umfasst und eine auf dem Scharnierachsenkern angeordnete oder gelagerte Scharnierachsenhülse. So kann beispielsweise der Scharnierachsenkern aus einem anderen Material gebildet sein als die Scharnierachsenhülse. Damit lässt sich eine Gleitpaarung zwischen beispielsweise der Scharnierachsenhülse und dem zweiten Gelenkelement optimieren und gleichzeitig eine Stabilität des Scharniergelenks. So kann die Scharnierachsenhülse insbesondere aus einem Kunststoff, beispielsweise Polytetrafluorethylen (PTFE), ausgebildet sein, der Scharnierachsenkern aus einem biokompatiblen Metall, beispielsweise einem Instrumentenstahl.

Vorzugsweise definieren die Scharnierachsenhülse und die die Scharnierachsenaufnahme das Gleitlager. Es kann also eine optimale Gleitpaarung ausgebildet werden zwischen der Scharnierachsenhülse und der Scharnierachsenaufnahme, wobei die Scharnierachsenhülse insbesondere aus einem geeigneten Kunststoff ausgebildet sein kann.

Besonders stabil ausbilden lässt sich die Kniegelenkendoprothese, wenn der Scharnierachsenkern in der Verbindungsstellung mit dem mindestens einen zweiten Verbindungselement kraft- und/oder formschlüssig in Eingriff steht. Insbesondere kann der Scharnierachsenkern das mindestens eine erste Verbindungselement umfassen. Beispielsweise können freie Enden des Scharnierachsenkerns jeweils ein erstes Verbindungselement bilden oder definieren.

Günstig ist es, wenn der Scharnierachsenkern einen kreisförmigen, ovalen oder einen eckigen, insbesondere einen vier- oder sechseckigen, Querschnitt aufweist. Insbesondere bei einem nicht kreisförmigen Querschnitt des Scharnierachsenkerns lässt sich eine verdrehsichere Verbindung zwischen dem Scharnierachsenkern und dem zweiten Verbindungselement und damit zwischen dem ersten Gelenkelement und der Femurkomponente ausbilden.

Ferner ist es vorteilhaft, wenn die Scharnierachsenhülse eine Scharnierachsenkernaufnahme aufweist zum Aufnehmen des Scharnierachsenkerns und wenn die Scharnierachsenkernaufnahme einen zum Scharnierachsenkern korrespondierenden freien Querschnitt aufweist. Ist der freie Querschnitt der Scharnierachsenkernaufnahme unrund wie beispielsweise ein ovaler oder eckiger Querschnitt eines Scharnierachsenkerns, so lässt sich eine drehfeste Verbindung zwischen der Scharnierachsenhülse und dem Scharnierachsenkern auf einfache Weise realisieren.

Günstigerweise steht der Scharnierachsenkern beidseits aus der Scharnierachsenhülse vor. Dies ermöglicht es insbesondere, aus der Scharnierachsenhülse vorstehende Abschnitte des Scharnierachsenkerns als erste Verbindungselemente zu nutzen zum Koppeln oder Verbinden des Scharnierachsenkerns und damit des ersten Gelenkelements mit der Femurkomponente.

Für eine einfache Kopplung des ersten Gelenkelements mit der Femurkomponente ist es vorteilhaft, wenn der Scharnierachsenkern beidseits aus der Scharnierachsenaufnahme vorsteht. Damit steht der Scharnierachsenkern also insbesondere beidseits aus dem zweiten Gelenkelement vor.

Um den Scharnierachsenkern mindestens teilweise zu schützen und eine optimale Gleitpaarung insbesondere auch mit anderen Bereichen oder Flächen der Femurkomponente realisieren zu können, ist es vorteilhaft, wenn die Scharnierachsenhülse beidseits aus der Scharnierachsenaufnahme vorsteht.

Gemäß einer weiteren bevorzugten Ausführungsform der Erfindung kann vorgesehen sein, dass die Femurkomponente eine Gelenkaufnahme zum Aufnehmen des Scharniergelenks aufweist und dass die zwei zweiten Verbindungselemente in aufeinander zu weisenden Wandflächen der Gelenkaufnahme ausgebildet sind. Die aufeinander zu weisenden Wandflächen können insbesondere an Wandabschnitten der Femurkomponente ausgebildet sein, die parallel zueinander ausgerichtet sind und auf ihren von den beiden Wandflächen weg weisenden Seitenflächen mit präparierten Flächen des Femurs in Kontakt stehen, an den die Femurkomponente der Kniegelenkendoprothese festgelegt wird. Die Gelenkaufnahme bietet die Möglichkeit, die Kniegelenkendoprothese möglichst kompakt auszubilden und das Scharniergelenk zudem auch schützend anzuordnen.

Auf einfache Weise ausbilden lässt sich die Verbindungseinrichtung, wenn die Verbindungsaufnahme in Form einer Nut ausgebildet ist mit einer parallel zur Verbindungsrichtung verlaufenden Nutlängsachse. So lässt sich ein erstes Verbindungselement auf einfache Weise in die Verbindungsaufnahme parallel zur Nutlängsachse einschieben.

Um das erste Gelenkelement und die Femurkomponente auf einfache Weise miteinander in Eingriff bringen zu können, ist es günstig, wenn die Einführöffnung eine seitliche Öffnung der Nut bildet. So muss also das erste Verbindungselement nicht von vorne in Richtung auf einen Nutboden der Nut eingeführt werden, sondern kann parallel zum Nutboden durch die Einführöffnung in die Nut eingeschoben werden.

Um eine definierte Positionierung des ersten Gelenkelements an der Femurkomponente sicherstellen zu können, ist es insbesondere günstig, wenn das mindestens eine zweite Verbindungselement einen in Richtung der Verbindungsrichtung wirksamen ersten Anschlag für das mindestens eine erste Verbindungselement in der Verbindungsstellung umfasst.

Auf einfache Weise ausbilden lässt sich die Verbindungseinrichtung, wenn der erste Anschlag eine in Richtung auf die Einführöffnung der Verbindungsaufnahme hin weisende erste Anschlagfläche umfasst. Die erste Anschlagfläche kann insbesondere eben oder gekrümmt ausgebildet sein. Insbesondere kann sie an eine äußere Kontur des ersten Gelenkelements, beispielsweise ein freies Ende der Scharnierachse oder des Scharnierachsenkerns, angepasst sein. Insbesondere kann eine formschlüssige Verbindung zwischen der Verbindungsaufnahme und der ersten Anschlagfläche einerseits und dem zweiten Verbindungselement erreicht werden.

Gemäß einer weiteren bevorzugten Ausführungsform der Erfindung kann vorgesehen sein, dass die Kniegelenkendoprothese eine Sicherungseinrichtung zum kraft- und/oder formschlüssigen Sichern der Verbindungseinrichtung in einer Sicherungsstellung umfasst, wenn die Verbindungseinrichtung die Verbindungsstellung einnimmt. Mit der Sicherungseinrichtung soll also insbesondere verhindert werden, dass die Verbindungseinrichtung von der Verbindungsstellung in unbeabsichtigter Weise in die Montagestellung überführt wird. Die Sicherungseinrichtung kann dabei eine Sicherungsstellung einnehmen, und zwar dann, wenn die Verbindungseinrichtung die Verbindungsstellung einnimmt, also die ersten und zweiten Verbindungselemente miteinander so in Eingriff stehen, dass die Femurkomponente mit dem ersten Gelenkelement gekoppelt ist. Die Sicherungseinrichtung kann insbesondere in Form einer Schraubverbindungseinrichtung ausgebildet sein. Eine Wirkrichtung der Schraubverbindungseinrichtung kann insbesondere quer, vorzugsweise senkrecht, oder parallel zur Verbindungsrichtung verlaufen.

Auf einfache Weise ausbilden lässt sich die Sicherungseinrichtung, wenn sie mindestens ein Sicherungselement umfasst zum kraft- und/oder formschlüssigen Sichern des mindestens einen ersten und des mindestens einen zweiten Verbindungselements in der Sicherungsstellung, wenn diese die Verbindungsstellung einnehmen. Mit dem mindestens einen Sicherungselement lässt sich also verhindern, dass die miteinander in Eingriff stehenden ersten und zweiten Verbindungselemente selbsttätig in unerwünschter Weise von der Verbindungsstellung in die Montagestellung übergehen. Das mindestens eine Sicherungselement kann insbesondere ein Innen oder Außengewinde umfassen zum Sichern des mindestens einen ersten und des mindestens einen zweiten Verbindungselements in der Sicherungsstellung. Beispielsweise kann das Sicherungselement in Form einer Sicherungsschraube mit einem Außengewindeabschnitt ausgebildet sein, die in eine Bohrung oder ein Sackloch mit korrespondierendem Innengewinde eingeschraubt werden kann, welche das mindestens eine erste und das mindestens eine zweite Verbindungselement mindestens teilweise durchsetzen, wenn diese die Verbindungsstellung einnehmen. Eine Längsachse des Sicherungselements kann insbesondere quer, vorzugsweise senkrecht, oder parallel zur Verbindungsrichtung verlaufen.

Besonders einfach und kompakt ausbilden lässt sich die Kniegelenkendoprothese, wenn das mindestens eine Sicherungselement in Form eines Verschlusselements ausgebildet ist zum Verschließen der Einführöffnung der Verbindungsaufnahme in der Sicherungsstellung. Mit dem Verschlusselement wird also lediglich die Einführöffnung der Verbindungsaufnahme verschlossen. Dadurch wird verhindert, dass das erste Verbindungselement wieder durch die Einführöffnung aus der Verbindungsaufnahme austreten kann.

Vorteilhaft ist es, wenn die Sicherungseinrichtung eine Rast- oder Schnappverbindungseinrichtung umfasst zum definierten Festlegen des mindestens einen Sicherungselements am mindestens einen ersten oder am mindestens einen zweiten Verbindungselement. Mit einer solchen Rast- oder Schnappverbindungseinrichtung kann beispielsweise das mindestens eine Sicherungselement mit dem ersten oder dem zweiten Verbindungselement, insbesondere mit der Verbindungsaufnahme an der Femurkomponente, in Eingriff gebracht werden, wobei durch die Rast- oder Schnappverbindungseinrichtung automatisch das mindestens eine Sicherungselement in der Sicherungsstellung definiert festgelegt ist.

Das mindestens eine Sicherungselement lässt sich auf einfache Weise an einem der Verbindungselemente festlegen, wenn die Rast- oder Schnappverbindungseinrichtung mindestens ein erstes Rastglied und mindestens ein mit diesem zusammenwirkendes zweites Rastglied umfasst, welche in einer Raststellung kraft- und/oder formschlüssig in Eingriff stehen und in einer Entsicherungsstellung außer Eingriff stehen, und wenn das mindestens eine Rastglied am mindestens einen Sicherungselement und wenn das mindestens eine zweite Rastglied am mindestens einen ersten oder am mindestens einen zweiten Verbindungselement angeordnet oder ausgebildet ist. Eine derart ausgebildete Rast- oder Schnappverbindungseinrichtung ermöglicht ein automatisches Verrasten oder Einschnappen der zusammenwirkenden ersten und zweiten Rastglieder, wenn das Sicherungselement zum Sichern der Verbindungseinrichtung in der Verbindungsstellung positioniert wird.

Um das automatische in Eingriff Bringen der ersten und zweiten Rastglieder zu verbessern, ist es vorteilhaft, wenn das mindestens eine erste und das mindestens eine zweite Rastglied in der Raststellung unter Vorspannung in Eingriff stehen. Stehen sie außer Eingriff, sind sie zwangsläufig ebenfalls gegeneinander vorgespannt, so dass sie, sobald dies möglich ist, automatisch die Raststellung einnehmen.

Um beispielsweise ein erstes Gelenkelement in Form einer Scharnierachse beidseitig in der Verbindungsstellung zu sichern, ist es günstig, wenn die Kniegelenkendoprothese zwei Sicherungselemente umfasst. Denkbar ist es auch, die beiden Sicherungselemente einstückig auszubilden, so dass nur ein einziges Sicherungselement erforderlich ist, um die Kniegelenkendoprothese in der Verbindungsstellung zu sichern.

Vorzugsweise umfasst das mindestens eine Sicherungselement einen in Richtung der Verbindungsrichtung wirksamen zweiten Anschlag für das mindestens eine erste Verbindungselement in der Verbindungsstellung. Der zweite Anschlag stellt insbesondere sicher, dass das erste Verbindungselement nicht in unbeabsichtigter Weise aus der Verbindungsstellung herausbewegt werden kann, insbesondere in die Montagestellung.

Auf einfache Weise und definiert lässt sich das mindestens eine erste Verbindungselement in der Verbindungsstellung halten, wenn der zweite Anschlag eine in Richtung auf die erste Anschlagfläche hin weisende zweite Anschlagfläche umfasst. Beispielsweise kann das mindestens eine erste Verbindungselement so zwischen der ersten und der zweiten Anschlagfläche sowohl in der Verbindungsstellung als auch in der Sicherungsstellung gehalten werden.

Zum automatischen Sichern des mindestens einen Sicherungselements in der Sicherungsstellung, ist es günstig, wenn dem mindestens einen Sicherungselement mindestens ein vorspannendes Element zugeordnet ist, um die Rast- oder Schnappverbindungseinrichtung unter Vorspannung in der Raststellung zu halten. Beispielsweise kann das vorspannende Element am mindestens einen Sicherungselement oder aber auch am mindestens einen ersten oder zweiten Verbindungselement angeordnet oder ausgebildet sein.

Besonders einfach und kostengünstig herstellen lässt sich die Kniegelenkendoprothese, wenn das mindestens eine vorspannende Element in Form eines Federelements ausgebildet ist. Insbesondere kann es in Form einer Blattfeder ausgebildet sein.

Mit besonders wenigen Teilen lässt sich die Kniegelenkendoprothese ausbilden, wenn das mindestens eine Sicherungselement das mindestens eine vorspannende Element umfasst. Das Sicherungselement kann insbesondere aus einem Metall oder aber auch aus einem körperverträglichen Kunststoff ausgebildet sein.

Die Herstellung der Kniegelenkendoprothese lässt sich weiter vereinfachen, wenn das mindestens eine Sicherungselement und das mindestens eine vorspannende Element einstückig ausgebildet sind.

Ferner kann eine vorspannende Kraft auf einfache Weise auf das mindestens eine erste oder das mindestens eine zweite Rastglied übertragen werden, wenn das mindestens eine vorspannende Element das mindestens eine erste oder das mindestens eine zweite Rastglied trägt.

Auf einfache Weise lässt sich die Rast- oder Schnappverbindungseinrichtung ausbilden, wenn das mindestens eine erste Rastglied in Form eines Rastvorsprungs oder einer Rastausnehmung ausgebildet ist und wenn das mindestens eine zweite Rastglied korrespondierend zum mindestens einen ersten Rastglied ausgebildet ist. Beispielsweise kann der Rastvorsprung in Form einer Rastnase am vorspannenden Element ausgebildet sein, welcher in der Sicherungsstellung in eine Rastausnehmung, beispielsweise in Form eines Rücksprungs am ersten Verbindungselement, insbesondere an der Verbindungsausnehmung, eingreift.

Um das mindestens eine Sicherungselement besonders gut in der Sicherungsstellung zu sichern, ist es vorteilhaft, wenn die Kniegelenkendoprothese zwei erste und zwei zweite Rastglieder aufweist. Insbesondere kann jedem vorspannenden Element ein Rastglied zugeordnet sein.

Gemäß einer bevorzugten Ausführungsform der Erfindung kann vorgesehen sein, dass das mindestens eine erste und das mindestens eine zweite Rastglied relativ zueinander von der Entsicherungsstellung in die Raststellung in einer Sicherungsrichtung bewegbar sind und umgekehrt und dass die Sicherungsrichtung quer zur Verbindungsrichtung und quer zur Drehachse verläuft. Insbesondere kann die Sicherungsrichtung senkrecht zur Verbindungsrichtung und senkrecht zur Drehachse verlaufen. Eine solche Ausgestaltung stellt sicher, dass beispielsweise in Richtung der Verbindungsrichtung vom ersten Gelenkelement ausgeübte Kräfte nicht zum außer Eingriff Bringen der Rast- oder Schnappverbindungseinrichtung geeignet sind, da hierfür noch eine Kraftkomponente parallel zur Sicherungsrichtung erforderlich wäre. Insbesondere kann also mit der vorgeschlagenen Weiterbildung erreicht werden, dass sich die miteinander gekoppelten Teile der Kniegelenkendoprothese auch nach der Implantation nicht selbstständig wieder voneinander trennen.

Auch wenn es grundsätzlich möglich ist, die Tibiakomponente und die Femurkomponente direkt miteinander zu verbinden, kann es ferner vorteilhaft sein, wenn die Kniegelenkendoprothese eine zwischen der Tibiakomponente und der Femurkomponente angeordnete, an der Femurkomponente oder der Tibiakomponente gehaltene Meniskuskomponente umfasst. Insbesondere kann die Meniskuskomponente unbeweglich oder beweglich an der Femurkomponente oder der Tibiakomponente gelagert sein. Die Meniskuskomponente weist zur Nachbildung eines natürlichen Knies üblicherweise zu Femurkondylen der Femurkomponente korrespondierende Gleitlagerflächen auf. Des Weiteren kann die Meniskuskomponente eine mit der Tibiakomponente zusammenwirkende Gleitlagerfläche aufweisen, wenn die Meniskuskomponente beispielsweise beweglich an der Tibiakomponente gelagert ist. Insbesondere kann sie verschiebbar und/oder rotierbar an der Tibiakomponente gelagert sein.

Um eine Längsrotation der Tibiakomponente relativ zur Femurkomponente in Folge einer Beugung, also einer Flexion des Knies, zu ermöglichen, ist es vorteilhaft, wenn die Meniskuskomponente und die Tibiakomponente um eine erste Rotationsachse relativ zueinander rotierbar gelagert sind.

Des Weiteren kann es aus diesem Grund auch vorteilhaft sein, wenn die Femurkomponente und die Tibiakomponente relativ zueinander um eine zweite Rotationsachse rotierbar gelagert sind.

Besonders kompakt ausbilden lässt sich die Kniegelenkendoprothese, wenn die erste und die zweite Rotationsachse identisch sind. Die Kniegelenkendoprothese kann insbesondere also derart ausgebildet sein, dass die Femurkomponente und die Tibiakomponente um dieselbe mathematische Achse relativ zueinander verdrehbar sind wie die Meniskuskomponente und die Tibiakomponente relativ zueinander.

Vorzugsweise ist das zweite Gelenkelement an der Tibiakomponente um die zweite Rotationsachse rotierbar gelagert. Damit kann über eine Relativbewegung des zweiten Gelenkelements und der Tibiakomponente auch die Femurkomponente, die über ein Scharniergelenk mit der Tibiakomponente gekoppelt ist, relativ zur Tibiakomponente um die zweite Rotationsachse rotiert oder verdreht werden.

Um eine einfache Kopplung des Scharniergelenks mit der Tibiakomponente zu ermöglichen, ist es vorteilhaft, wenn das zweite Gelenkelement einen Gelenkzapfen umfasst, welcher die erste und/oder die zweite Rotationsachse definiert und in eine Gelenkzapfenaufnahme der Tibiakomponente eingreift.

Um eine Rotation insbesondere um eine Längsachse der Tibia zu ermöglichen, ist es vorteilhaft, wenn der Gelenkzapfen in der Gelenkzapfenaufnahme rotierbar und/oder verschiebbar gehalten ist. Eine Verschiebbarkeit ermöglicht es insbesondere, auch ein Abheben der Femurkomponente von der Tibiakomponente und gegebenenfalls auch von der Meniskuskomponente in einem begrenzten oder auch unbegrenzten Umfang zu gestatten.

Vorteilhaft ist es ferner, insbesondere auch bei einer Kniegelenkendoprothese der eingangs beschriebenen Art, wenn sie eine Luxationssicherungseinrichtung zum Verhindern eines außer Eingriff Bringens des zweiten Gelenkelements und der Tibiakomponente umfasst. Mit der Luxationssicherungseinrichtung lässt sich also insbesondere verhindern, dass die Femurkomponente und die Tibiakomponente, die miteinander gekoppelt sind, in undefinierter Weise voneinander entkoppelt werden. Die Luxationssicherungseinrichtung setzt nicht zwingend voraus, dass die Verbindungsrichtung quer zur Drehachse verläuft.

Günstig ist es insbesondere, wenn die Luxationssicherungseinrichtung mindestens einen Luxationssicherungsanschlag umfasst zum Begrenzen einer Bewegung des zweiten Gelenkelements und der Tibiakomponente relativ zueinander parallel zur ersten und/oder zweiten Rotationsachse. Es kann sich dabei um einen Luxationssicherungsanschlag handeln, welcher eine Bewegung des zweiten Gelenkelements in Richtung auf die Tibiakomponente hin begrenzt und/oder von der Tibiakomponente weg.

Vorteilhaft ist es, wenn die Luxationssicherungseinrichtung zwei Luxationssicherungsanschläge umfasst zum Begrenzen einer Bewegung des zweiten Gelenkelements und der Tibiakomponente aufeinander zu und voneinander weg. Mit zwei Luxationssicherungsanschlägen ist es also möglich, insbesondere das zweite Gelenkelement und die Tibiakomponente unlösbar miteinander zu koppeln, jedoch eine Relativbewegung zwischen beiden Teilen und damit auch zwischen der Femurkomponente und der Tibiakomponente relativ zueinander in einem begrenzten Umfang zu ermöglichen, und zwar aufeinander zu und voneinander weg.

Gemäß einer weiteren bevorzugten Ausführungsform der Erfindung kann vorgesehen sein, dass die Luxationssicherungseinrichtung mindestens ein erstes Verriegelungselement und mindestens ein zweites Verriegelungselement umfasst, die in einer Verriegelungsstellung in Eingriff stehen und in einer Freigabestellung außer Eingriff stehen, dass das mindestens eine erste Verriegelungselement dem zweiten Gelenkelement zugeordnet oder an diesem angeordnet oder ausgebildet ist und dass das mindestens eine zweite Verriegelungselement der Tibiakomponente zugeordnet oder an dieser angeordnet oder ausgebildet ist. Mit einer solchen Luxationssicherungseinrichtung ist es insbesondere möglich, das zweite Gelenkelement und die Tibiakomponente in der Verriegelungsstellung definiert miteinander zu koppeln. Insbesondere kann eine solche Kopplung bereits vor der Implantation der Tibiakomponente erfolgen, da das erste Gelenkelement und das zweite Gelenkelement optional auch nach unabhängiger Implantation der Femurkomponente und der Tibiakomponente miteinander verbunden werden können zum Koppeln derselben.

Um das mindestens eine erste Verriegelungselement und das mindestens eine zweite Verriegelungselement miteinander in Eingriff zu bringen, ist es günstig, wenn diese relativ zueinander bewegbar angeordnet oder ausgebildet sind. Insbesondere können sie in einer Verriegelungsrichtung quer zur zweiten Rotationsachse relativ zueinander bewegbar angeordnet oder ausgebildet sein. Eine solche Bewegbarkeit quer zur zweiten Rotationsachse kann auf sichere Weise eine Bewegung des zweiten Gelenkelements und der Tibiakomponente parallel zur zweiten Rotationsachse verhindern.

Des Weiteren ist es vorteilhaft, wenn die Luxationssicherungseinrichtung ein Blockierelement umfasst zum Verhindern einer Bewegung des mindestens einen ersten Verriegelungselements und des mindestens einen zweiten Verriegelungselements relativ zueinander von der Verriegelungsstellung in die Freigabestellung. Mit dem Blockierelement lässt sich also die Kniegelenkendoprothese in der Verriegelungsstellung sichern, so dass ein Trennen des zweiten Gelenkelements von der Tibiakomponente in Abhängigkeit der Ausgestaltung der Kniegelenkendoprothese insbesondere nicht möglich ist.

Um eine Relativbewegung insbesondere der Femurkomponente und der Tibiakomponente parallel zur zweiten Rotationsachse zumindest in einem begrenzten Umfang zu ermöglichen, ist es günstig, wenn das mindestens eine erste Verriegelungselement und das mindestens eine zweite Verriegelungselement in der Verriegelungsstellung relativ zueinander in einer Richtung parallel zur zweiten Rotationsachse bewegbar sind. Insbesondere kann eine Bewegung der Verriegelungselemente relativ zueinander durch entsprechende Anschläge mit zusammenwirkenden Anschlagflächen verhindert oder eingeschränkt werden.

Vorteilhaft ist es, wenn das mindestens eine erste Verriegelungselement in Form eines beweglich gelagerten Verriegelungskörpers ausgebildet ist und wenn das mindestens eine zweite Verriegelungselement in Form einer Verriegelungsaufnahme ausgebildet ist, in welcher der Verriegelungskörper in der Verriegelungsstellung eingreift. Beispielsweise kann der beweglich gelagerte Verriegelungskörper am zweiten Gelenkelement oder aber auch direkt oder indirekt an der Tibiakomponente angeordnet oder ausgebildet sein. Indirekt bedeutet beispielsweise, dass ein weiteres, an der Tibiakomponente festgelegtes Bauteil der Kniegelenkendoprothese den Verriegelungskörper oder die Verriegelungsaufnahme umfasst. Die Verriegelungsaufnahme kann insbesondere in Form einer in Richtung auf die erste und/oder zweite Rotationsachse hin weisend geöffneten, umlaufenden Ringnut ausgebildet sein.

Günstigerweise ist der Verriegelungskörper in Form einer Kugel ausgebildet, welche in einer quer zur zweiten Rotationsachse verlaufenden Kugelbohrung beweglich gehalten ist. Insbesondere kann die Kugelbohrung am zweiten Gelenkelement, beispielsweise am Gelenkzapfen, oder aber auch an der Tibiakomponente oder einer an dieser gehaltenen weiteren Komponente beweglich gehalten sein. Insbesondere können zwei, drei oder mehr erste und/oder zweite Verriegelungselemente vorgesehen sein, beispielsweise drei Kugeln mit entsprechenden Verriegelungsaufnahmen, in die die Kugel aus der Kugelbohrung vorstehend eingreifen können.

Auf besonders einfache Weise lässt sich das Blockierelement in Form eines Bolzens ausbilden, welcher in der Verriegelungsstellung in eine parallel oder im Wesentlichen parallel zur zweiten Längsachse verlaufenden Bolzenaufnahme des zweiten Gelenkelements eingreift und eine Bewegung des mindestens einen ersten Verriegelungselements in Richtung auf die erste und/oder die zweite Rotationsachse hin blockiert. Insbesondere kann die Bolzenaufnahme am Gelenkzapfen ausgebildet sein. Diese Ausgestaltung ermöglicht es insbesondere, die Femurkomponente und die Tibiakomponente unabhängig voneinander zu Implantieren und durch in Eingriff Bringen des zweiten Gelenkelements mit der Gelenkelementaufnahme in der Tibiakomponente zu koppeln, wobei die Verbindung zwischen der Tibiakomponente und der Femurkomponente durch das in den Gelenkzapfen eingreifende Blockierelement sicherbar ist.

Für die Handhabung der Kniegelenkendoprothese bei der Implantation ist es günstig, wenn die Bolzenaufnahme in Form eines Sacklochs ausgebildet ist, welches in Richtung auf die Femurkomponente hin weisend geöffnet ist. Diese Ausgestaltung ermöglicht es insbesondere, das Blockierelement in die Bolzenaufnahme einzuschieben in einer Richtung parallel zur ersten und/oder zweiten Rotationsachse, um so die Femurkomponente und die Tibiakomponente miteinander zu koppeln. Insbesondere ist es bei einem Blockierelement, wie es oben beschrieben wurde, nicht erforderlich, am Bolzen einen Verriegelungskonus vorzusehen, welcher mit einem korrespondierenden Verriegelungskonus an der Bolzenaufnahme zusammenwirkt, um die Femurkomponente mit der Tibiakomponente zu koppeln. Ein Sackloch hat zudem den Vorteil, dass das Blockierelement nicht in Richtung auf die Femurkomponente hin über den Gelenkzapfen vorstehen und diesen verlängern muss.

Um zu verhindern, dass sich das Blockierelement unbeabsichtigt löst und dadurch die Luxationssicherungseinrichtung unbeabsichtigt in die Freigabestellung überführt wird, ist es günstig, wenn der Bolzen in die Bolzenaufnahme einschraubbar ausgebildet ist. Ein solcher einschraubbarer Bolzen löst sich in der Praxis praktisch nicht aus der Bolzenaufnahme.

Wie bereits erwähnt, muss das mindestens eine zweite Verriegelungselement nicht direkt an der Tibiakomponente angeordnet oder ausgebildet sein. Günstig ist es, wenn das mindestens eine zweite Verriegelungselement an einer Verriegelungshülse angeordnet oder ausgebildet ist und wenn die Verriegelungshülse in der Gelenkzapfenaufnahme kraft- und/oder formschlüssig gehalten ist. Eine solche Verriegelungshülse vorzusehen hat insbesondere den Vorteil, dass das mindestens eine zweite Verriegelungselement in unterschiedlichen Positionen anordenbar ist. Insbesondere lässt sich so die Kniegelenkendoprothese modular ausbilden und je nach Patient eine Position des mindestens einen zweiten Verriegelungselements an der Tibiakomponente individuell einstellen. Zudem kann man so wahlweise auch eine Verschiebbarkeit des zweiten Gelenkelements in der Gelenkzapfenaufnahme individuell vorgeben. Dafür muss dann nicht die gesamte Tibiakomponente entsprechend ausgebildet werden, sondern es kann mit einer Standardtibiakomponente und einer Auswahl unterschiedlicher Verriegelungshülsen die für einen Patienten optimale Kniegelenkendoprothese ausgebildet werden.

Günstigerweise ist die Verriegelungshülse mit einem Rückhalteelement in der Gelenkzapfenaufnahme gehalten. Das Rückhaltelement dient also insbesondere dazu, die Verriegelungshülse zumindest axial an der Femurkomponente festzulegen. Dies bedeutet, dass die Verriegelungshülse nicht oder nur eingeschränkt parallel zur ersten und/oder zweiten Rotationsachse bewegbar ist.

Auf einfache Weise lässt sich die Kniegelenkendoprothese ausbilden, wenn das Rückhalteelement in Form einer Rückhaltehülse und die Verriegelungshülse umgebend mit der Gelenkzapfenaufnahme verschraubbar ausgebildet ist. Beispielsweise kann die Rückhaltehülse in Form einer Überwurfmutter oder eines einschraubbaren Einsatzes ausgebildet sein.

Gemäß einer weiteren bevorzugten Ausführungsform der Erfindung kann vorgesehen sein, dass die Gelenkzapfenaufnahme einen ersten Verriegelungshülsenanschlag definiert und dass das Rückhalteelement einen zweiten Verriegelungshülsenanschlag definiert und dass die Verriegelungshülse durch den ersten und den zweiten Verriegelungshülsenanschlag in der Gelenkzapfenaufnahme gehalten ist. Die Verriegelungshülsenanschläge verhindern also insbesondere eine Bewegung der Verriegelungshülse in der Gelenkzapfenaufnahme parallel zur ersten und/oder zweiten Rotationsachse. Sie können insbesondere so ausgebildet sein, dass die Verriegelungshülse in der Gelenkzapfenaufnahme nicht verschiebbar ist oder so, dass sie eingeschränkt verschiebbar ist.

Vorteilhaft ist es, wenn die Femurkomponente und die Tibiakomponente in der Montagestellung vollständig voneinander getrennt sind und wenn sie nach einer unabhängigen Implantation der Femurkomponente und der Tibiakomponente voneinander ausgehend von der Montagestellung miteinander koppelbar sind durch Koppeln des mindestens einen ersten und des mindestens einen zweiten Verbindungselements miteinander. Diese Ausgestaltung ermöglicht eine individuelle Implantation von Femurkomponente und Tibiakomponente, wobei Einschränkungen im Falle einer Kopplung der beiden Komponenten bereits vor der Implantation nicht gegeben sind.

Um eine definierte Rotation der Meniskuskomponente relativ zur Tibiakomponente zu ermöglichen, ist es vorteilhaft, wenn die Tibiakomponente ein Drehlagerelement aufweist zum rotierbaren Lagern der Meniskuskomponente um die erste Rotationsachse.

Vorzugsweise ist das erste Drehlagerelement in Form eines von der Tibiakomponente abstehenden Zapfens ausgebildet. Dieser Zapfen kann insbesondere hülsenförmig ausgebildet sein und sich in die Gelenkzapfenaufnahme hinein verlängern. So lässt sich die Kniegelenkendoprothese besonders kompakt ausbilden.

Die nachfolgende Beschreibung bevorzugter Ausführungsformen der Erfindung dient im Zusammenhang mit den Zeichnungen der näheren Erläuterung. Es zeigen:

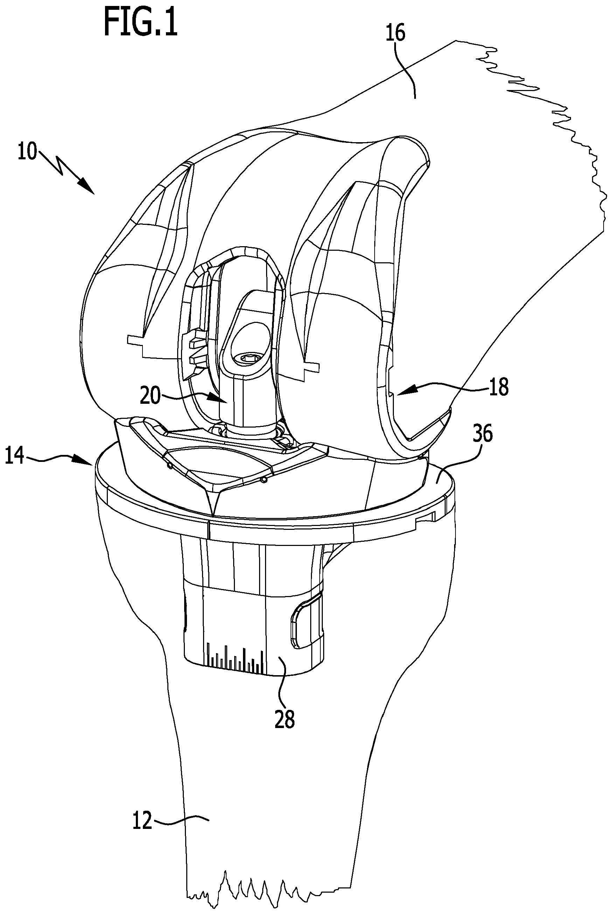

- Figur 1:: eine perspektivische Ansicht eines Ausführungsbeispiels einer an einem Femur und an einer Tibia festgelegten Kniegelenkendoprothese;

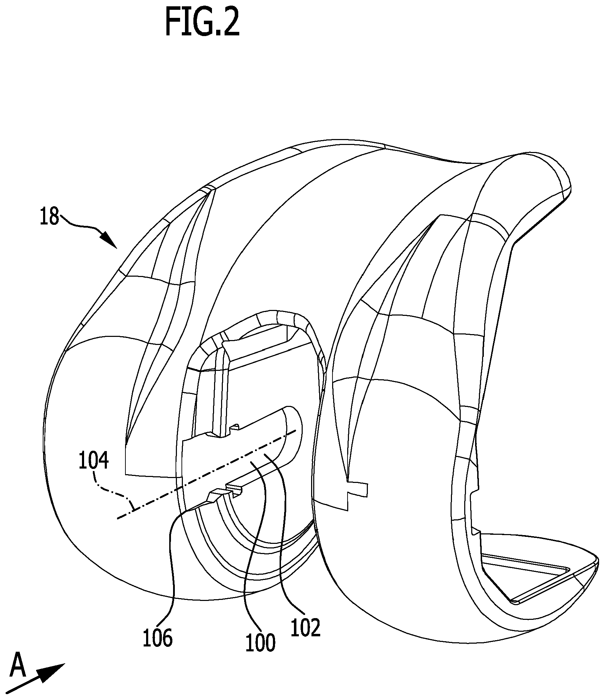

- Figur 2:: eine perspektivische Ansicht der Femurkomponente der Kniegelenkendoprothese aus Figur 1;

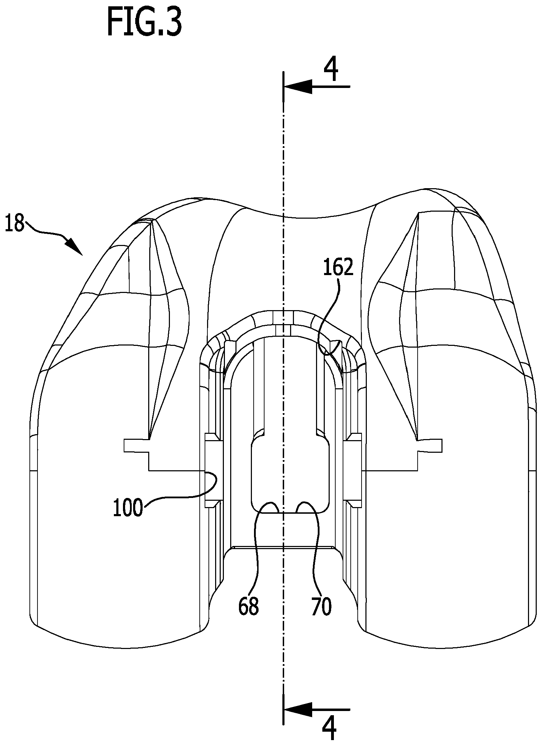

- Figur 3:: eine Ansicht in Richtung des Pfeils A in Figur 2;

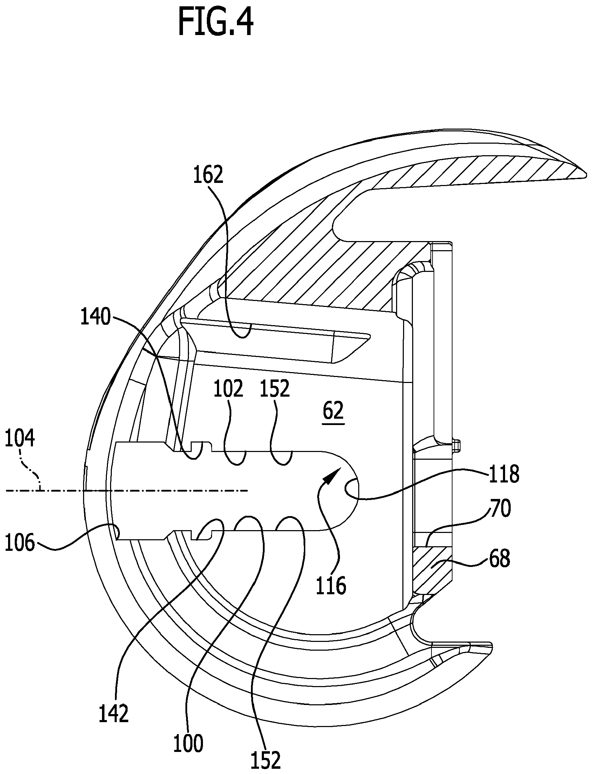

- Figur 4:: eine Schnittansicht längs Linie 4-4 in Figur 3;

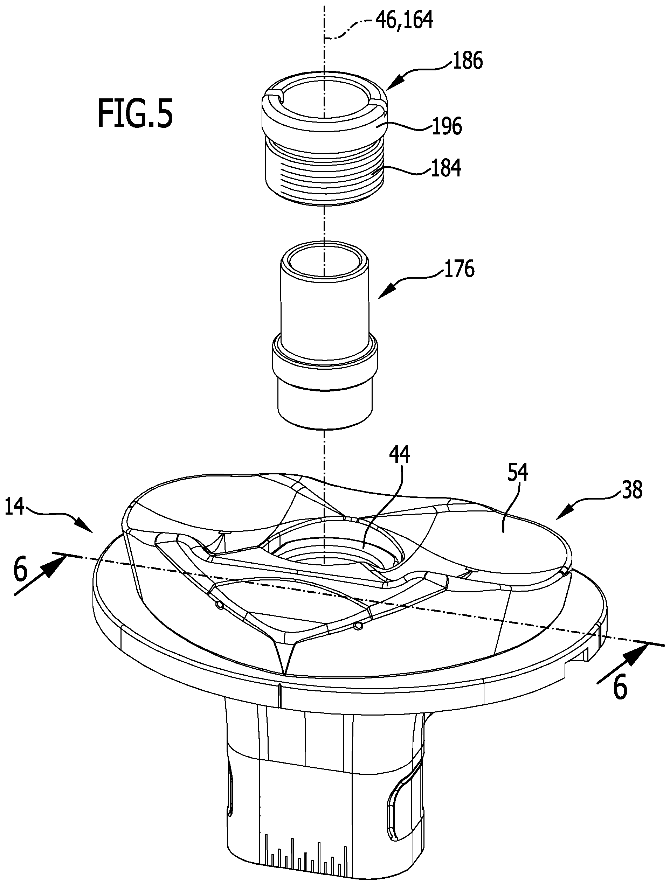

- Figur 5:: eine perspektivische Explosionsdarstellung der Tibiakomponente der Kniegelenkendoprothese aus Figur 1;

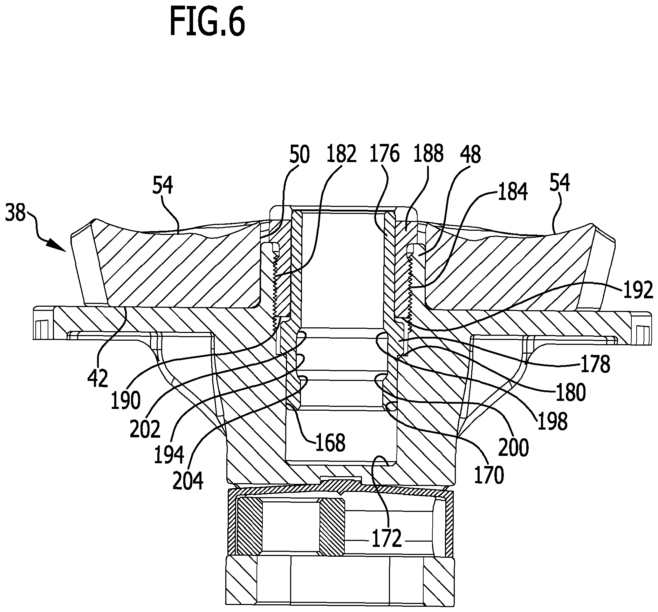

- Figur 6:: eine Schnittansicht längs Linie 6-6 in Figur 5;

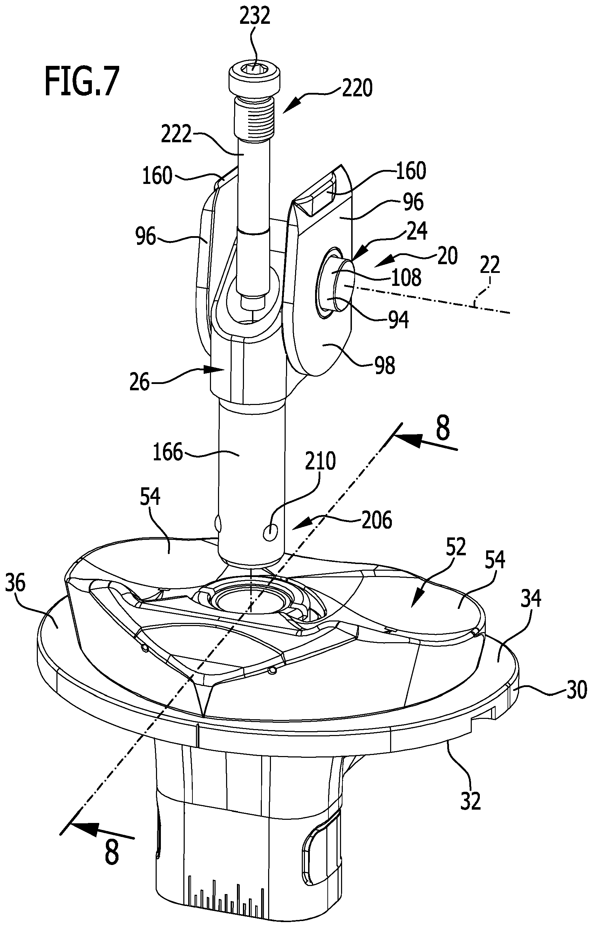

- Figur 7:: eine perspektivische, teilweise Explosionsdarstellung der Tibiakomponente aus Figur 5 mit Scharniergelenk;
- Figur 8:: eine Schnittansicht längs Linie 8-8 in Figur 7;
- Figur 9:: eine perspektivische Ansicht der Kniegelenkendoprothese aus Figur 1 vor dem Koppeln des ersten Gelenkelements und der Femurkomponente;

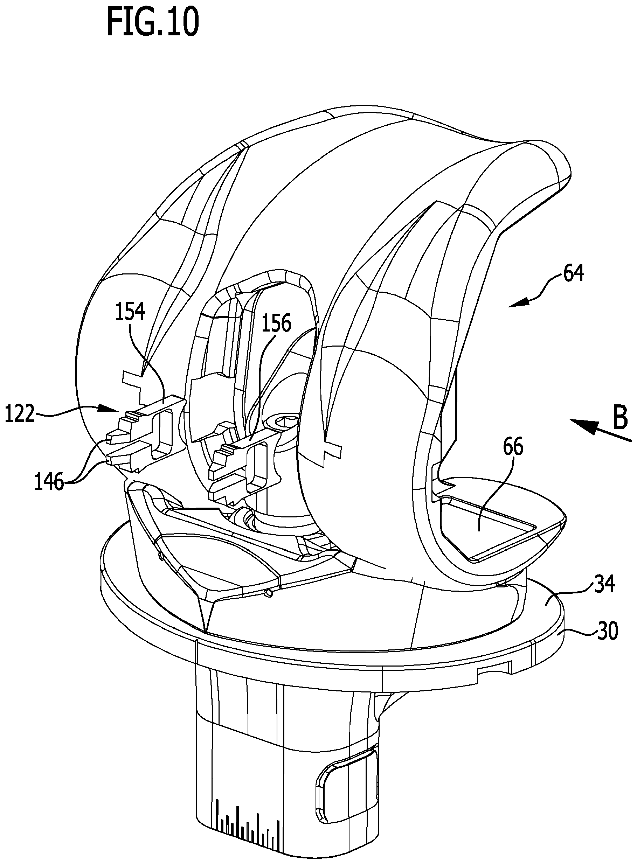

- Figur 10:: eine perspektivische Ansicht der Kniegelenkendoprothese aus Figur 1 nach dem Koppeln von Femurkomponente und Tibiakomponente beim Einsetzen von zwei Sicherungselementen;

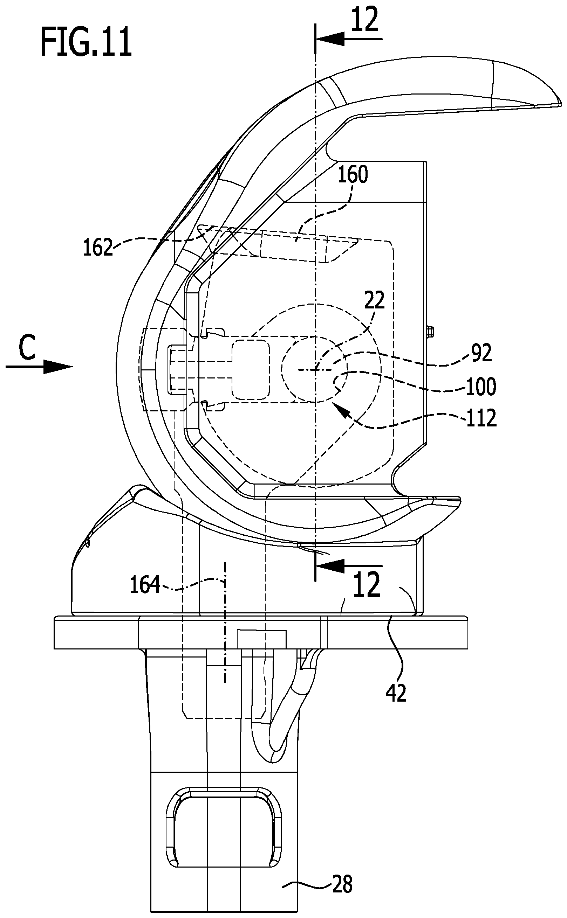

- Figur 11:: eine teilweise durchbrochene Ansicht der Anordnung aus Figur 10 in Richtung des Pfeils B;
- Figur 12:: eine Schnittansicht längs Linie 12-12 in Figur 11;

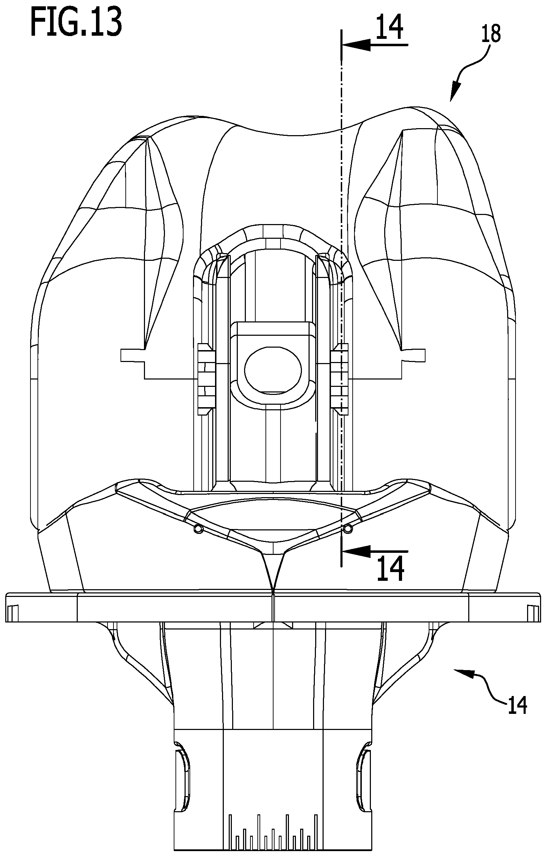

- Figur 13:: eine Ansicht der Anordnung aus Figur 11 in Richtung des Pfeils C; und
- Figur 14:: eine Schnittansicht längs Linie 14-14 in Figur 13.

In den Figuren ist schematisch ein Ausführungsbeispiel einer insgesamt mit dem Bezugszeichen 10 bezeichneten Kniegelenkendoprothese dargestellt. Sie umfasst eine an einer Tibia 12 festlegbare Tibiakomponente 14 und eine an einem Femur 16 festlegbare Femurkomponente 18.

Die Tibiakomponente und die Femurkomponente 18 sind nach der Implantation der Kniegelenkendoprothese 10 über ein Scharniergelenk 20 um eine Drehachse 22 drehgelenkig gekoppelt.

Das Scharniergelenk 20 umfasst ein erstes Gelenkelement 24 und ein mit diesem um die Drehachse 22 verdrehbar gekoppeltes zweites Gelenkelement 26.

Die Tibiakomponente 14 umfasst einen in eine Kavität der Tibia 12 einführbaren und darin festlegbaren Schaft 28. Dieser kann optional modular ausgebildet sein mit in den Figuren nicht dargestellten, unterschiedlich langen Verlängerungen, so dass die Tibiakomponente 14 abhängig von der Physiologie des Patienten optimal in dessen Tibia 12 verankert werden kann.

Die Tibiakomponente 14 umfasst ferner eine Platte 30, von deren Unterseite 32 sich der Schaft 28 senkrecht oder im Wesentlichen senkrecht weg erstreckt. Eine Oberseite 34 der Platte 30 bildet eine ebene Gelenkfläche 36, auf welcher eine optional von der Kniegelenkendoprothese 10 umfasste Meniskuskomponente mit ihrer Unterseite 40, die eine ebene Anlagefläche 42 definiert, aufliegt.

Die Meniskuskomponente 38 ist beweglich auf der Platte 30 gelagert. Hierfür weist die Tibiakomponente 14 ein Drehlagerelement 44 auf zum rotierbaren Lagern der Meniskuskomponente 38 um eine erste Rotationsachse 46. Diese wird definiert durch das erste Drehlagerelement 44, welches in Form eines von der Oberseite 34 senkrecht abstehenden Zapfens 48 ausgebildet ist. Dieser ragt in eine korrespondierend zu ihm ausgebildete Zapfenaufnahme 50 in Form einer Durchbrechung einer Meniskuskomponente 38 hinein. Auf diese Weise ist die Meniskuskomponente 38 um die erste Rotationsachse 46 verdrehbar auf der Platte 30 gelagert.

Die Meniskuskomponente 38 weist auf ihrer Oberseite 52 zwei Gelenkflächen 54 auf, an denen von der Femurkomponente 18 in Richtung auf die Tibiakomponente 14 hin weisende Kondylenflächen 56 anliegen und abgleiten, wenn die Femurkomponente 18 relativ zur Tibiakomponente 14 um die Drehachse 22 verdreht wird.

Die Femurkomponente 14 weist ferner eine boxartige Gelenkaufnahme 58 auf, die von zwei parallel zueinander verlaufenden Wänden 60 begrenzt wird. Die Wände 60 weisen aufeinander zu weisende Wandflächen 62 auf, die senkrecht zur Drehachse 22 verlaufen.

Des Weiteren ist eine Rückseite 64 der Femurkomponente 18 mit einer Mehrzahl von Knochenanlageflächen 66 mit Vertiefungen in Form von Zementtaschen versehen, um die Femurkomponente 18 an präparierte Knochenflächen des Femurs 16 anzulegen und zu fixieren, insbesondere mit Knochenzement.

Optional kann die Femurkomponente 18 mit einem in den Figuren nicht dargestellten Schaft gekoppelt werden. Hierfür ist an einer weiteren, die Gelenkaufnahme 58 begrenzenden, quer zu den Wänden 16 verlaufenden Wand 68 eine Durchbrechung 70 ausgebildet, die von einem Verbindungselement durchsetzt oder mit einem Verlängerungsschaft zum Einsetzen in eine Kavität des Femurs 16 gekoppelt werden kann.

Das erste Gelenkelement 24 definiert mit seiner Längsachse die Drehachse 22. Es ist in Form einer Scharnierachse 72 ausgebildet, welches eine korrespondierende Scharnierachsenaufnahme 74 des zweiten Gelenkelements 26 durchsetzt. Die Scharnierachsenaufnahme 74 ist in Form einer Durchgangsbohrung 76 an einem der Femurkomponente 18 zugewandten Endabschnitt 78 des zweiten Gelenkelements 26 ausgebildet. Die Scharnierachse 72 und die Scharnierachsenaufnahme 74 bilden ein Gleitlager 80.

Die Scharnierachse 72, die insgesamt die Form eines langgestreckten Kreiszylinders aufweist, umfasst einen Scharnierachsenkern 82 und eine diesen umgebende Scharnierachsenhülse 84. Damit definieren eine Außenfläche 86 der Scharnierachsenhülse 84 ebenso wie eine Innenfläche 88 der Scharnierachsenaufnahme 74 Gleitflächen des Gleitlagers 80.

Um eine möglichst gute, verschleißarme Gleitpaarung auszubilden, können so die Materialien des zweiten Gelenkelements 26 und der Scharnierachsenhülse 84 optimal aufeinander abgestimmt werden. Beispielsweise kann die Scharnierachsenhülse 84 aus einem Kunststoff ausgebildet sein, das zweite Gelenkelement 26 aus einem Instrumentenstahl.

Bei dem in den Figuren dargestellten Ausführungsbeispiel weist der Scharnierachsenkern 82 einen kreisförmigen Querschnitt auf. Die Scharnierachsenhülse 84 kann über eine Presssitzpassung mit dem Scharnierachsenkern 82 gekoppelt sein.

Alternativ kann der Scharnierachsenkern auch einen unrunden Querschnitt, beispielsweise einen ovalen oder einen eckigen, insbesondere einen vier- oder sechseckigen, Querschnitt, aufweisen. Ein Querschnitt einer Scharnierachsenkernaufnahme 90 der Scharnierhülse 84 weist dann vorzugsweise einen korrespondierenden Querschnitt auf, so dass die Scharnierachsenhülse 84 drehfest mit dem Scharnierachsenkern 82 verbunden ist, wenn der Querschnitt des Scharnierachsenkerns 82 unrund ist.

Bei dem in den Figuren dargestellten Ausführungsbeispiel steht der Scharnierachsenkern 82 beidseits aus der Scharnierachsenhülse 84 vor. Freie Enden 92 des Scharnierachsenkerns 82 bilden auf diese Weise Verbindungsvorsprünge 94.

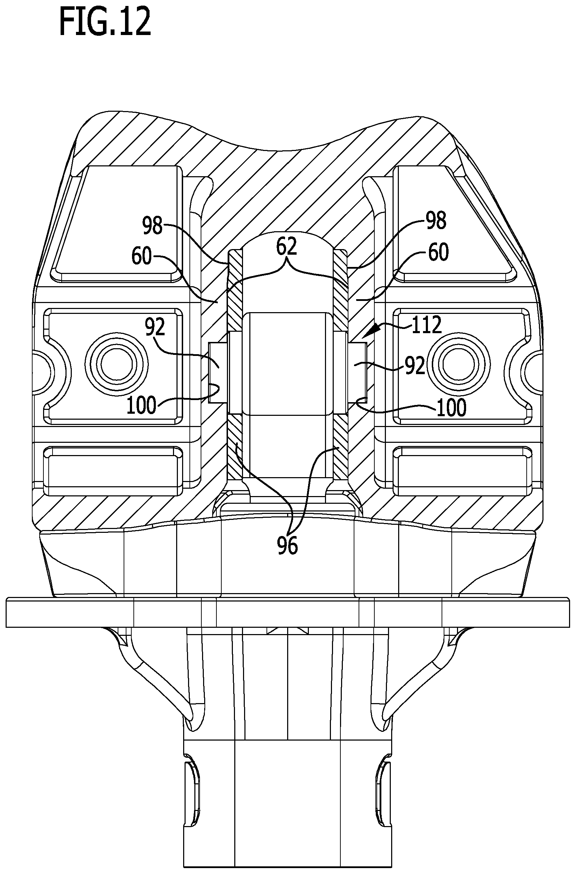

Wie beispielsweise in Figur 12 gut zu erkennen, ragt der Scharnierachsenkern 82 auch beidseits aus der Scharnierachsenaufnahme 74 heraus.

Des Weiteren steht auch die Scharnierachsenhülse 84 beidseits aus der Scharnierachsenaufnahme 74 vor.

Auf der aus der Scharnierachsenaufnahme 74 vorstehenden Scharnierachsenhülse 84 sitzt beidseits des Endabschnitts 78 eine Gleitlagerplatte 96. Diese kann insbesondere aus demselben Material gebildet sein wie die Scharnierachsenhülse 84. Voneinander weg weisende Seitenflächen 98 der Gleitlagerplatten 96 liegen an den Wandflächen 62 an.

Die Gelenkaufnahme 58 weist in den Wandflächen jeweils eine Verbindungsaufnahme 100 auf, die in Form einer Nut 102 ausgebildet ist, die eine Nutlängsachse 104 definiert, die quer, insbesondere senkrecht, zur Drehachse 22 verläuft.

Die Nut ist an ihrem der Wand 68 abgewandten Ende offen und definiert eine Einführöffnung 106.

Die Verbindungsvorsprünge 94 bilden erste Verbindungselemente 108, die Verbindungsaufnahmen 100 zweite Verbindungselemente 110 einer insgesamt mit dem Bezugszeichen 112 bezeichneten Verbindungseinrichtung zum Verbinden des ersten Gelenkelements 24 mit der Femurkomponente 18.

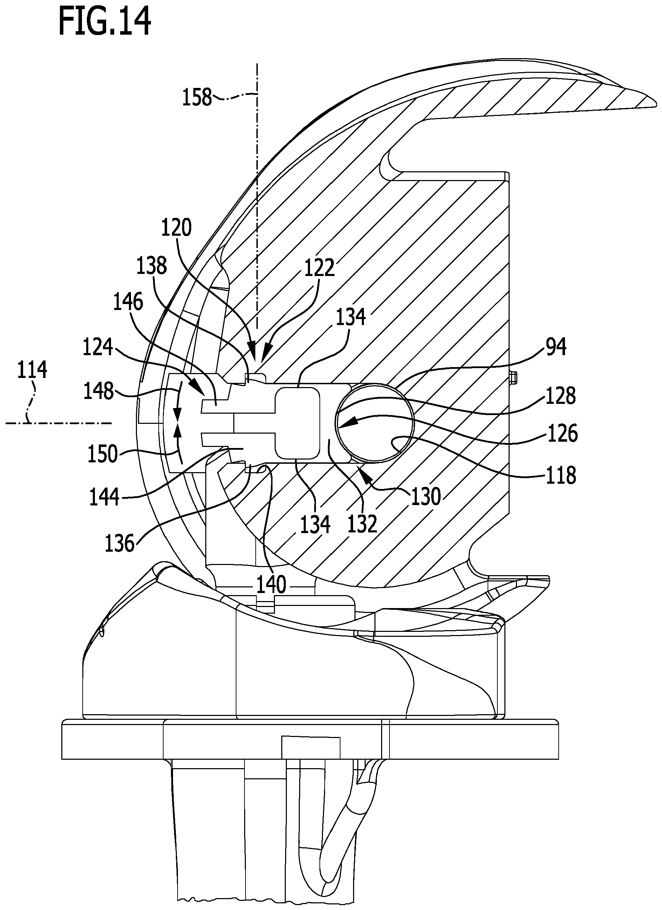

Die Verbindungseinrichtung 112 ist derart ausgebildet, dass sie eine Verbindungsstellung definiert, in welcher die ersten Verbindungselemente 108 und die zweiten Verbindungselemente 110 kraft- und/oder formschlüssig in Eingriff stehen. Diese Verbindungsstellung ist beispielsweise in den Figuren 12 und 14 schematisch dargestellt.

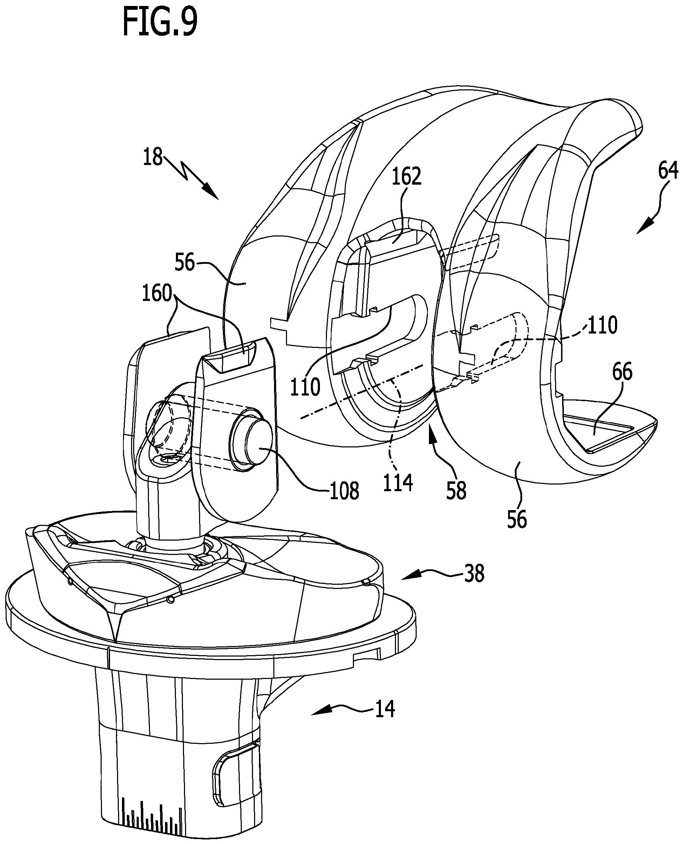

Ferner definiert die Verbindungseinrichtung 112 eine Montagestellung, in welcher das erste Gelenkelement 24 und die Femurkomponente 18 vollständig voneinander getrennt sind. Die Verbindungselemente 108 und 110 stehen in dieser Stellung außer Eingriff. Die Montagestellung ist beispielsweise in Figur 9 dargestellt.

Die ersten Verbindungselemente 108 sind dem ersten Gelenkelement 24 zugeordnet beziehungsweise an diesem angeordnet oder ausgebildet. Die zweiten Verbindungselemente 110 sind der Femurkomponente 18 zugeordnet beziehungsweise an dieser angeordnet oder ausgebildet.

Die Verbindungseinrichtung 112 definiert ferner eine Verbindungsrichtung 114, in welcher die ersten Verbindungselemente 108 und die zweiten Verbindungselemente 110 relativ zueinander bewegbar sind zum Überführen der Verbindungseinrichtung 112 von der Montagestellung in die Verbindungsstellung. Die Verbindungsrichtung wird definiert durch die Nutlängsachse 104, denn die Verbindungsvorsprünge 94 können nur parallel zur Nutlängsachse 104 in die Verbindungsaufnahmen 110 eingeschoben werden. Damit verläuft die Verbindungsrichtung 114 aber auch quer, nämlich senkrecht zur Drehachse 22.

Durch die besondere Ausgestaltung der Kniegelenkendoprothese 10 steht in der Verbindungsstellung der Scharnierachsenkern 82 mit den zweiten Verbindungselementen 110 kraft- und/oder formschlüssig in Eingriff.

Die zweiten Verbindungselemente 110 weisen einen in Richtung der Verbindungsrichtung 114 wirksamen ersten Anschlag 116 für die ersten Verbindungselemente 108 in der Verbindungsstellung auf. Der erste Anschlag 116 wird gebildet durch eine erste Anschlagfläche 118, welche in Richtung auf die Einführöffnung 106 der Verbindungsaufnahme 100 hin weist. In der Verbindungsstellung liegt, wie in Figur 14 schematisch dargestellt, der Verbindungsvorsprung 94 an der ersten Anschlagfläche 118 an.

Um die miteinander in Eingriff stehenden ersten und zweiten Verbindungselemente 108, 110 in der Verbindungsstellung zu sichern, ist eine Sicherungseinrichtung 120 zum kraft- und/oder formschlüssigen Sichern der Verbindungseinrichtung 112 in einer Sicherungsstellung, die beispielhaft in Figur 14 dargestellt ist, vorgesehen, wenn die Verbindungseinrichtung 112 die Verbindungsstellung einnimmt. Die Sicherungseinrichtung 120 umfasst zwei Sicherungselemente 122 zum kraft- und formschlüssigen Sichern der ersten und zweiten Verbindungselemente 108, 110 in der Sicherungsstellung, wenn diese die Verbindungsstellung einnehmen.

Die Sicherungselemente 122 sind in Form von Verschlusselementen 124 ausgebildet zum Verschließen der Einführöffnung 106 der Verbindungsaufnahme 100 in der Sicherungsstellung. Die Verschlusselemente sind im Wesentlichen quaderförmig ausgebildet und umfassen einen zweiten Anschlag 126 für das zweite Verbindungselement 110. Der zweite Anschlag 126 umfasst eine zweite Anschlagfläche 128, die in Richtung auf die erste Anschlagfläche 118 hin weist. Wie in Figur 14 gut zu erkennen, wird das erste Verbindungselement 108 zwischen den beiden Anschlagflächen 118 und 128 in der Verbindungsstellung gehalten.

Damit die Sicherungselemente 122 in Folge einer auf die Scharnierachse 72 wirkenden Kraft nicht aus der Verbindungsaufnahme 100 austreten können, umfasst die Sicherungseinrichtung 120 eine Rast- oder Schnappverbindungseinrichtung 130 zum definierten Festlegen der Sicherungselemente 122 am zweiten Verbindungselement 110.

Das Sicherungselement 122 umfasst einen im Wesentlichen quaderförmigen Anschlagkörper 132, an welchem die zweite Anschlagfläche 128 ausgebildet ist.

Parallel zur Verbindungsrichtung 114 erstrecken sich vorspannende Elemente 134, die die Rast- oder Schnappverbindungseinrichtung 130, nachfolgend nur noch als Rastverbindungseinrichtung bezeichnet, unter Vorspannung in der in Figur 14 dargestellten Raststellung halten.

Die Rastverbindungseinrichtung 130 umfasst erste Rastglieder 136, die in Form von Rastvorsprüngen 138 ausgebildet sind, die in korrespondierende Rastausnehmungen 140, die zweite Rastglieder 142 definieren, in der Raststellung eingreifen. Wie in Figur 14 zu erkennen, stehen die ersten und zweiten Rastglieder 136, 142 in der Raststellung kraft- und/oder formschlüssig in Eingriff.

Die Vorsprünge 138 sind jeweils an einem Träger 144 angeordnet, von welchen parallel zum vorspannenden Element 134 vom Träger 144 in Richtung auf die Einführöffnung 106 hin weisende Betätigungsvorsprünge abstehen. Werden die beiden Betätigungsvorsprünge 146 in Richtung der Pfeile 148 und 150 aufeinander zu bewegt, geben die Rastvorsprünge 138 die Rastausnehmungen 140 frei, so dass die Rastverbindungseinrichtung 130 eine in den Figuren nicht dargestellte Entsicherungsstellung einnimmt.

Die ersten Rastglieder 136 sind somit am Sicherungselement 122 angeordnet, die zweiten Rastglieder an den zweiten Verbindungselementen 110 ausgebildet. Die Rastausnehmungen 140 weisen aufeinander zu und sind in aufeinander zu weisenden Nutseitenwänden 152 der Nut 102 ausgebildet.

Die vorspannenden Elemente 134 sind in Form von Federelementen 154 ausgebildet, und zwar als Blattfedern 156. Diese halten die Rastverbindungseinrichtung 130 in der Raststellung unter Vorspannung. Bei dem in den Figuren dargestellten Ausführungsbeispiel umfasst das Sicherungselement 122 zwei vorspannende Elemente 134 und ist vorzugsweise einstückig mit diesen ausgebildet. Das Sicherungselement 122 kann insbesondere aus einem Metall oder einem Kunststoff gebildet sein.

Durch die Anordnung der Rastvorsprünge 138 auf den Trägern 144 trägt auch jedes vorspannende Element 134 jeweils ein erstes Rastglied 136.

Des Weiteren ist die Rastverbindungseinrichtung 130 derart ausgebildet, dass die ersten und zweiten Rastglieder 136 und 142 relativ zueinander von der Entsicherungsstellung in die Raststellung in einer Sicherungsrichtung 158 bewegbar sind und umgekehrt. Die Sicherungsrichtung 158 verläuft quer zur Verbindungsrichtung 114 und quer zur Drehachse 22. Bei dem in den Figuren dargestellten Ausführungsbeispiel der Kniegelenkendoprothese 10 verlaufen die Sicherungsrichtung 158, die Drehachse 22 und die Verbindungsrichtung 114 jeweils senkrecht zueinander.

Um die Gleitlagerplatten 96 rotationsgesichert in der Gelenkaufnahme 58 zu halten, ist an jeder Gleitlagerplatte 96 ein Vorsprung 160 in Richtung auf die Wandfläche 62 hin weisend ausgebildet, der in der Verbindungsstellung in eine korrespondierende Ausnehmung 162 in den Wänden 60 eingreift.

Die Kniegelenkendoprothese 10 ist ferner derart ausgebildet, dass die Femurkomponente 18 und die Tibiakomponente 14 relativ zueinander um eine zweite Rotationsachse 164 rotierbar oder verdrehbar gelagert sind. Bei dem in den Figuren dargestellten Ausführungsbeispiel sind die erste und die zweite Rotationsachse 46, 164 identisch.

Das zweite Gelenkelement 26 ist an der Tibiakomponente 14 um die zweite Rotationsachse 164 rotierbar gelagert. Wie dies erreicht wird, wird nachfolgend noch im Einzelnen erläutert.

Das zweite Gelenkelement 26 umfasst einen vom Endabschnitt 78 abstehenden und einstückig mit diesem ausgebildeten Gelenkzapfen 166. Dieser definiert die erste und die zweite Rotationsachse 46, 164 und greift in eine Gelenkzapfenaufnahme 168 der Tibiakomponente 14 ein. Die Gelenkzapfenaufnahme 168 ist in Form eines Sacklochs 170 ausgebildet und an ihrem von der Femurkomponente 18 weg weisenden Ende verschlossen durch einen Sacklochboden 172.

Um die Femurkomponente 18 und die Tibiakomponente 14 relativ zueinander um die Rotationsachsen 46 und 164 verdrehen zu können, ist der Gelenkzapfen 166 in der Gelenkzapfenaufnahme 168 rotierbar gelagert.

Des Weiteren ist der Gelenkzapfen 166 in der Gelenkzapfenaufnahme 168 bei dem in den Figuren dargestellten Ausführungsbeispiel auch parallel zu den Rotationsachsen 46 und 164 verschiebbar gehalten. Letzteres wird insbesondere erreicht durch eine Luxationssicherungseinrichtung 174. Diese verhindert ein außer Eingriff Bringen des zweiten Gelenkelements 26 und der Tibiakomponente 14.

Wie nachfolgend noch im Einzelnen beschrieben wird, ist das zweite Gelenkelement 26 mit der Tibiakomponente 14 koppelbar und kann an dieser auch dauerhaft gehalten werden.

In die Gelenkzapfenaufnahme 168 ist eine Verriegelungshülse 176 eingesetzt, die einen in radialer Richtung von der Rotationsachse 46 weg weisenden Ringvorsprung trägt. Der Ringvorsprung 178 ist in die Gelenkzapfenaufnahme 168 so weit einschiebbar, bis er an einen ersten Verriegelungshülsenanschlag 180 anschlägt, der durch eine einstufige Querschnittsverjüngung der Gelenkzapfenaufnahme 168 gebildet ist.

Das Drehlagerelement 44 begrenzt ebenfalls die Gelenkzapfenaufnahme 168 und ist somit hülsenförmig ausgebildet. Ausgehend von einem in Richtung auf die Femurkomponente 18 hin weisenden Ende des Drehlagerelements 44 ist dieses mit einem Innengewindeabschnitt 182 versehen, welcher zu einem Außengewindeabschnitt 184 einer Rückhaltehülse 186 korrespondiert. Ein am der Femurkomponente 18 zugewandten Ende der Rückhaltehülse 186 ausgebildeter, in radialer Richtung von der Rotationsachse 46 weg weisender Ringvorsprung 188 bildet einen Tiefenanschlag für das Einschrauben der Rückhaltehülse 186 in die Gelenkzapfenaufnahme 168.

Eine von der Femurkomponente 18 weg weisende ringförmige Endfläche 190 der Rückhaltehülse 186 bildet einen zweiten Verriegelungshülsenanschlag 192 für den Ringvorsprung 178. Damit ist eine Bewegung der Verriegelungshülse 176 in Richtung auf die Femurkomponente 18 hin durch den zweiten Verriegelungsanschlag 192 begrenzt, in Richtung in die Gelenkzapfenaufnahme 168 hinein durch den ersten Verriegelungshülsenanschlag 180.

An der Verriegelungshülse 176 ist etwa in Höhe des Ringvorsprungs 178 in ihrem Innern eine Ringnut 194 ausgebildet, die in Richtung auf die Rotationsachse 46 hin geöffnet ist.

Die Rückhaltehülse 186 bildet ein Rückhalteelement 196 zum Halten der Verriegelungshülse 176 in der Gelenkzapfenaufnahme 168.

Die Luxationssicherungseinrichtung 174 umfasst einen ersten Luxationssicherungsanschlag 198 und einen zweiten Luxationssicherungsanschlag 200 zum Begrenzen einer Bewegung des zweiten Gelenkelements 26 und der Tibiakomponente 14 relativ zueinander parallel zu den Rotationsachsen 46 und 164. Die Luxationssicherungsanschläge 198, 200 werden gebildet durch im Wesentlichen aufeinander zu weisende Nutseitenflächen 202 und 204 der Ringnut 194. Die Nutseitenfläche 202 begrenzt dabei eine Bewegung des zweiten Gelenkelements 26 von der Tibiakomponente 14 weg, die Nutseitenfläche 204 eine Bewegung des zweiten Gelenkelements 26 in die Gelenkzapfenaufnahme 168 hinein.

Ferner umfasst die Luxationssicherungseinrichtung 174 erste und zweite Verriegelungselemente 206 und 208, die in einer Verriegelungsstellung in Eingriff stehen und in einer Freigabestellung außer Eingriff.

Bei dem in den Figuren dargestellten Ausführungsbeispiel sind drei erste Verriegelungselemente 206 und ein zweites Verriegelungselement 208 vorgesehen. Die ersten Verriegelungselemente 206 sind dabei dem zweiten Gelenkelement 26 zugeordnet beziehungsweise an diesem angeordnet und das zweite Verriegelungselement 208 ist der Tibiakomponente 14 zugeordnet beziehungsweise an dieser angeordnet oder ausgebildet.

Die ersten Verriegelungselemente 206 sind in Form beweglich gelagerter Verriegelungskörper 210 ausgebildet. Das zweite Verriegelungselement 208 ist in Form einer Verriegelungsaufnahme 212 ausgebildet.

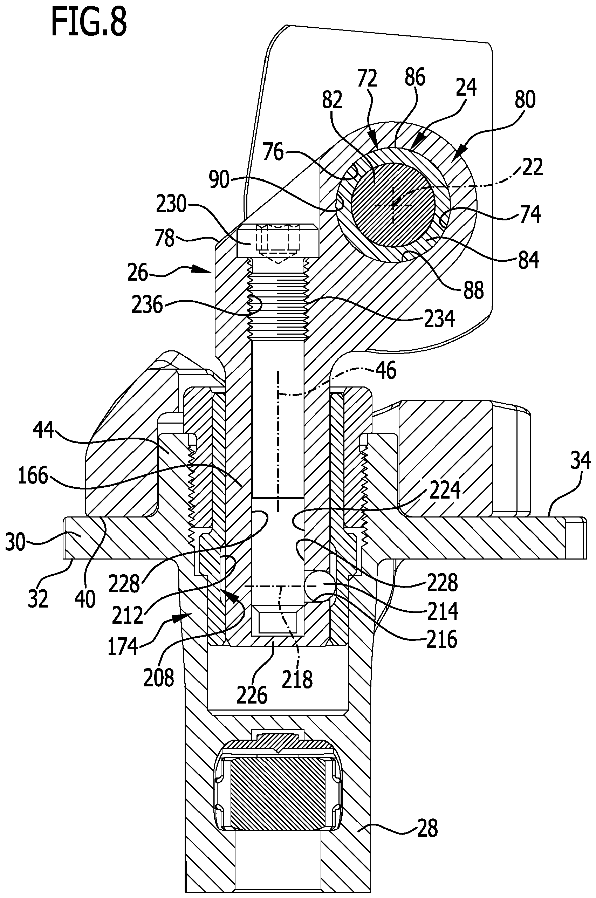

In einer Verriegelungsstellung, wie sie beispielhaft in Figur 8 dargestellt ist, greift der Verriegelungskörper 210 in die Verriegelungsaufnahme 212 ein. Die Verriegelungsaufnahme 212 wird gebildet durch die Ringnut 194. Jeder Verriegelungskörper 210 ist in Form einer Kugel 214 ausgebildet, welche in einer quer zur zweiten Rotationsachse 164 verlaufenden Kugelbohrung 216 beweglich gehalten ist.

Ein Durchmesser der Kugel 214 ist kleiner als ein Abstand der Nutseitenflächen 202 und 204 voneinander, sodass die ersten Verriegelungselemente 206 und das zweite Verriegelungselement 208 in der Verriegelungsstellung, in welcher die Verriegelungselemente 206 und 208 in Eingriff stehen, in einer Richtung parallel zur zweiten Rotationsachse 164 bewegbar sind.

Die Verriegelungselemente 206 und 208 sind relativ zueinander bewegbar wie beschrieben, und zwar in einer Verriegelungsrichtung 218, die quer zur zweiten Rotationsachse 164 verläuft. Insbesondere können die Kugeln 214 in den Kugelbohrungen 216 in radialer Richtung von der Rotationsachse 164 weg oder auf diese hin bewegt werden.

Des Weiteren umfasst die Luxationssicherungseinrichtung 174 ein Blockierelement 220 zum Verhindern einer Bewegung der ersten Verriegelungselemente 206 und des zweiten Verriegelungselements 208 relativ zueinander von der Verriegelungsstellung in eine Freigabestellung, in welcher sie außer Eingriff stehen.

Das Blockierelement 220 ist in Form eines Bolzens 222 ausgebildet, welcher in der Verriegelungsstellung in eine parallel oder im Wesentlichen parallel zur zweiten Rotationsachse 164 verlaufende Bolzenaufnahme 224 des zweiten Gelenkelements 26 eingreift. Die Bolzenaufnahme 224 erstreckt sich in den Gelenkzapfen 160 hinein. Dieser ist an seinem von der Femurkomponente 18 weg weisenden Enden 226 verschlossen, sodass der Bolzen 222 den Gelenkzapfen 166 nicht verlängern kann.

Die Bolzenaufnahme 224 ist in Form eines Sacklochs 228 ausgebildet, welches in Richtung auf die Femurkomponente 18 hin weisend geöffnet ist.

Der Bolzen 222 weist an seinem der Femurkomponente 18 zugewandten Ende einen Kopf 230 mit einer Werkzeugelementaufnahme 232 in Form eines Innenvielkant oder Innenvielrund auf. An den Kopf 230 schließt sich ein Außengewindeabschnitt 234 an, welcher zu einem Innengewindeabschnitt 236 des Sacklochs 228 ausgehend von dessen in Richtung auf die Femurkomponente 18 hin weisenden Ende korrespondiert. Durch diese Ausgestaltung ist der Bolzen 222 in die Bolzenaufnahme 224 einschraubbar.

Im Bereich seines distalen Endes füllt der Bolzen 222 die Bolzenaufnahme 224 im Querschnitt aus, sodass die Kugeln 214 an einer Bewegung in Richtung auf die zweite Rotationsachse 164 gehindert sind.

Die Kniegelenkendoprothese 10 ermöglicht es einem Operateur, zunächst die Tibiakomponente 14 und die Femurkomponente 18 unabhängig voneinander an der Tibia 12 beziehungsweise dem Femur 16 festzulegen. Vor der Implantation der Tibiakomponente 14 wird das Scharniergelenk 20 mit der Tibiakomponente 14 gekoppelt. Dazu wird der Gelenkzapfen 166 in die Gelenkzapfenaufnahme 168 eingeführt und durch Einschrauben des Bolzens 222 in die Bolzenaufnahme 224 der Luxationssicherungseinrichtung in die Verriegelungsstellung überführt, in welcher die Kugeln 214 in die Ringnut 194 eingreifen.

Zum Koppeln der Femurkomponente 18 und der Tibiakomponente 14 miteinander werden nun die ersten und zweiten Verbindungselemente 108 und 110 miteinander in Eingriff gebracht, und zwar indem die Scharnierachse 72 in der bereits oben beschriebenen Weise in die Verbindungsaufnahmen 100 eingeschoben wird. Zum Sichern der Verbindungseinrichtung in der Verbindungsstellung werden abschließend die beiden Sicherungselemente 122 der Sicherungseinrichtung 120 in der beschriebenen Weise in die Verbindungsaufnahmen 100 eingeschoben, bis die Rastglieder 136 und 132 ineinander greifen.

Zur Anpassung der Kniegelenkendoprothese 10 an unterschiedliche Physiologien von Patienten können unterschiedlich hohe Meniskuskomponenten 38 vorgesehen werden. Um eine optimale Verbindung der Tibiakomponente 14 mit der Femurkomponente 18 zu erreichen, können dann entweder unterschiedliche zweite Gelenkelemente 26 mit unterschiedlich langen Gelenkzapfen 166 bereitgestellt werden und/oder aber unterschiedliche Verriegelungshülsen 176, bei denen die Ringnut 194 unterschiedlich im Abstand von einem in Richtung auf die Femurkomponente hin weisenden Ende der Verriegelungshülse 176 angeordnet ist. So lassen sich auf einfache Weise modulare Kniegelenkendoprothesen herstellen, wobei lediglich zusammengehörige Paare von Meniskuskomponenten 38 und Verriegelungshülsen 176 bereitgestellt werden müssen, um dem Operateur eine individuelle Anpassung der Kniegelenkendoprothese 10 an die jeweiligen Bedürfnisse eines Patienten zu gestatten.

### Bezugszeichenliste

- 10: Kniegelenkendoprothese
- 12: Tibia
- 14: Tibiakomponente
- 16: Femur
- 18: Femurkomponente
- 20: Scharniergelenk
- 22: Drehachse
- 24: erstes Gelenkelement
- 26: zweites Gelenkelement
- 28: Schaft
- 30: Platte
- 32: Unterseite
- 34: Oberseite
- 36: Gelenkfläche
- 38: Meniskuskomponente
- 40: Unterseite
- 42: Anlagefläche
- 44: Drehlagerelement
- 46: erste Rotationsachse
- 48: Zapfen
- 50: Zapfenaufnahme
- 52: Oberseite
- 54: Gelenkfläche
- 56: Kondylenfläche
- 58: Gelenkaufnahme
- 60: Wand
- 62: Wandfläche
- 64: Rückseite
- 66: Knochenanlagefläche
- 68: Wand
- 70: Durchbrechung
- 72: Scharnierachse
- 74: Scharnierachsenaufnahme
- 76: Durchgangsbohrung
- 78: Endabschnitt
- 80: Gleitlager
- 82: Scharnierachsenkern
- 84: Scharnierachsenhülse
- 86: Außenfläche
- 88: Innenfläche
- 90: Scharnierachsenaufnahme
- 92: freies Ende
- 94: Verbindungsvorsprung
- 96: Gleitlagerplatte
- 98: Seitenfläche
- 100: Verbindungsaufnahme
- 102: Nut
- 104: Nutlängsachse
- 106: Einführöffnung
- 108: erstes Verbindungselement
- 110: zweites Verbindungselement
- 112: Verbindungseinrichtung
- 114: Verbindungsrichtung
- 116: erster Anschlag
- 118: erste Anschlagfläche
- 120: Sicherungseinrichtung
- 122: Sicherungselement
- 124: Verschlusselement
- 126: zweiter Anschlag
- 128: zweite Anschlagfläche
- 130: Rast- oder Schnappverbindungseinrichtung
- 132: Anschlagkörper
- 134: vorspannendes Element
- 136: erstes Rastglied
- 138: Rastvorsprung
- 140: Rastausnehmung
- 142: zweites Rastglied
- 144: Träger
- 146: Betätigungsvorsprung
- 148: Pfeil
- 150: Pfeil
- 152: Nutseitenwand
- 154: Federelement
- 156: Blattfeder
- 158: Sicherungsrichtung
- 160: Vorsprung
- 162: Ausnehmung
- 164: zweite Rotationsachse
- 166: Gelenkzapfen
- 168: Gelenkzapfenaufnahme
- 170: Sackloch
- 172: Sacklochboden
- 174: Luxationssicherungseinrichtung
- 176: Verriegelungshülse
- 178: Ringvorsprung
- 180: erster Verriegelungshülsenanschlag
- 182: Innengewindeabschnitt
- 184: Außengewindeabschnitt
- 186: Rückhaltehülse
- 188: Ringvorsprung
- 190: Endfläche
- 192: zweiter Verriegelungshülsenanschlag
- 194: Ringnut
- 196: Rückhalteelement
- 198: erster Luxationssicherungsanschlag
- 200: zweiter Luxationssicherungsanschlag
- 202: Nutseitenfläche
- 204: Nutseitenfläche
- 206: erstes Verriegelungselement
- 208: zweites Verriegelungselement
- 210: Verriegelungskörper
- 212: Verriegelungsaufnahme
- 214: Kugel
- 216: Kugelbohrung
- 218: Verriegelungsrichtung
- 220: Blockierelement
- 222: Bolzen
- 224: Bolzenaufnahme
- 226: Ende
- 228: Sackloch
- 230: Kopf
- 232: Werkzeugelementaufnahme
- 234: Außengewindeabschnitt
- 236: Innengewindeabschnitt

## Patentansprüche

1. Kniegelenkendoprothese (10) mit einer Tibiakomponente (14) und einer Femurkomponente (18) und einem Scharniergelenk (20) zum drehgelenkigen Koppeln der Tibiakomponente (14) und der Femurkomponente (18) um eine Drehachse (22), welches Scharniergelenk ein erstes Gelenkelement (24) und ein mit diesem um die Drehachse (22) verdrehbar gekoppeltes zweites Gelenkelement (26) umfasst, wobei eine Verbindungseinrichtung (112) mit mindestens einem ersten Verbindungselement (108) und mindestens einem zweiten Verbindungselement (110) zum Verbinden des ersten Gelenkelements (24) mit der Femurkomponente (18) vorgesehen ist, welche Verbindungseinrichtung (112) eine Verbindungsstellung, in welcher das mindestens eine erste Verbindungselement (108) und das mindestens eine zweite Verbindungselement (110) kraft- und/oder formschlüssig in Eingriff stehen, und eine Montagestellung, in welcher das erste Gelenkelement (24) und die Femurkomponente (18) vollständig voneinander getrennt sind, definiert, wobei das mindestens eine erste Verbindungselement (108) dem ersten Gelenkelement (24) zugeordnet oder an diesem angeordnet oder ausgebildet ist und wobei das mindestens eine zweite Verbindungselement (110) der Femurkomponente (18) zugeordnet oder an dieser angeordnet oder ausgebildet ist, wobei die Verbindungseinrichtung (112) eine Verbindungsrichtung (114) definiert, in welcher das mindestens eine erste Verbindungselement (108) und das mindestens eine zweite Verbindungselement (110) relativ zueinander bewegbar sind zum Überführen der Verbindungseinrichtung (112) von der Montagestellung in die Verbindungsstellung, wobei die Verbindungsrichtung (114) quer, insbesondere senkrecht, zur Drehachse (22) verläuft, **dadurch gekennzeichnet, dass** das erste Gelenkelement (24) in Form einer Scharnierachse (72) ausgebildet ist und dass das zweite Gelenkelement (26) eine Scharnierachsenaufnahme (74) aufweist, die von der Scharnierachse (72) durchsetzt ist.

2. Kniegelenkendoprothese nach Anspruch 1, **dadurch gekennzeichnet, dass**
a) das zweite Gelenkelement (26) an der Tibiakomponente (14) gehalten oder mit dieser koppelbar ist
und/oder
b) die Verbindungseinrichtung (112) derart ausgebildet ist, dass sie nach einer unabhängigen Implantation der Femurkomponente (18) und der Tibiakomponente (14) voneinander von der Montagestellung in die Verbindungsstellung überführbar ist,
und/oder
c) eine vom ersten Gelenkelement (24) definierte Längsachse die Drehachse (22) definiert
und/oder
d) das mindestens eine erste Verbindungselement (108) in Form eines Verbindungsvorsprungs (94) und dass das mindestens eine zweite Verbindungselement (110) in Form einer Verbindungsaufnahme (100) ausgebildet ist oder umgekehrt,
wobei insbesondere sich die Verbindungsaufnahme (100) parallel zur Verbindungsrichtung (114) erstreckt und eine Einführöffnung (106) zum Einführen des Verbindungsvorsprungs (94) parallel zur Verbindungsrichtung (114) aufweist,
und/oder
e) die Kniegelenkendoprothese zwei erste und/oder zwei zweite Verbindungselemente (108, 110) umfasst.

3. Kniegelenkendoprothese nach einem der voranstehenden Ansprüche, **dadurch gekennzeichnet, dass**
a) die Scharnierachsenaufnahme (74) in Form einer Durchgangsbohrung (76) ausgebildet ist
und/oder
b) die Scharnierachse (72) und die Scharnierachsenaufnahme (74) ein Gleitlager (80) bilden.

4. Kniegelenkendoprothese nach einem der voranstehenden Ansprüche, **dadurch gekennzeichnet, dass** die Scharnierachse (72) einen Scharnierachsenkern (82) umfasst und eine auf dem Scharnierachsenkern (82) angeordnete oder gelagerte Scharnierachsenhülse (84).

5. Kniegelenkendoprothese nach Anspruch 4, **dadurch gekennzeichnet, dass**
a) die Scharnierachsenhülse (84) und die Scharnierachsenaufnahme (74) das Gleitlager (80) definieren
und/oder
b) der Scharnierachsenkern (82) in der Verbindungsstellung mit dem mindestens einen zweiten Verbindungselement (110) kraft- und/ oder formschlüssig in Eingriff steht
und/oder
c) der Scharnierachsenkern (82) einen kreisförmigen, ovalen oder einen eckigen, insbesondere einen vier- oder sechseckigen, Querschnitt aufweist
und/oder
d) die Scharnierachsenhülse (84) eine Scharnierachsenkernaufnahme (90) aufweist zum Aufnehmen des Scharnierachsenkerns und dass die Scharnierachsenkernaufnahme einen zum Scharnierachsenkern (82) korrespondierenden freien Querschnitt aufweist und/oder
e) der Scharnierachsenkern (82) beidseits aus der Scharnierachsenhülse (84) vorsteht
und/oder
f) der Scharnierachsenkern (82) beidseits aus der Scharnierachsenaufnahme (74) vorsteht
und/oder
g) die Scharnierachsenhülse (84) beidseits aus der Scharnierachsenaufnahme (74) vorsteht.

6. Kniegelenkendoprothese nach einem der voranstehenden Ansprüche, **gekennzeichnet durch** eine Sicherungseinrichtung (120) zum kraft- und/ oder formschlüssigen Sichern der Verbindungseinrichtung (112) in einer Sicherungsstellung, wenn die Verbindungseinrichtung (112) die Verbindungsstellung einnimmt, und dass die Sicherungseinrichtung (120) mindestens ein Sicherungselement (122) umfasst zum kraft- und/oder formschlüssigen Sichern des mindestens einen ersten und des mindestens einen zweiten Verbindungselements (108, 110) in der Sicherungsstellung, wenn diese die Verbindungsstellung einnehmen.

7. Kniegelenkendoprothese nach Anspruch 6, **dadurch gekennzeichnet, dass**
a) das mindestens eine Sicherungselement (122) in Form eines Verschlusselements (124) ausgebildet ist zum Verschließen der Einführöffnung (106) der Verbindungsaufnahme (100) in der Sicherungsstellung
und/oder
b) die Sicherungseinrichtung (120) eine Rast- oder Schnappverbindungseinrichtung (130) umfasst zum definierten Festlegen des mindestens einen Sicherungselements (122) am mindestens einen ersten oder am mindestens einen zweiten Verbindungselement (110), dass die Rast- oder Schnappverbindungseinrichtung (130) mindestens ein erstes Rastglied (136) und mindestens ein mit diesem zusammenwirkendes zweites Rastglied (142) umfasst, welche in einer Raststellung kraft- und/oder formschlüssig in Eingriff stehen und in einer Entsicherungsstellung außer Eingriff stehen, und dass das mindestens eine erste Rastglied (136) am mindestens einen Sicherungselement (122) und dass das mindestens eine zweite Rastglied (142) am mindestens einen ersten oder am mindestens einen zweiten Verbindungselement (110) angeordnet oder ausgebildet ist,
wobei insbesondere
b1) das mindestens eine erste und das mindestens eine zweite Rastglied (136, 142) in der Raststellung unter Vorspannung in Eingriff stehen
und/oder
b2) die Kniegelenkendoprothese zwei erste und zwei zweite Rastglieder (136, 142) umfasst
und/oder
b3) das mindestens eine erste und das mindestens eine zweite Rastglied (136, 142) relativ zueinander von der Entsicherungsstellung in die Raststellung in einer Sicherungsrichtung (158) bewegbar sind und umgekehrt und dass die Sicherungsrichtung (158) quer zur Verbindungsrichtung (114) und quer zur Drehachse (22) verläuft, insbesondere senkrecht zur Verbindungsrichtung (114) und senkrecht zur Drehachse (22),
und/oder
c) die Kniegelenkendoprothese zwei Sicherungselemente (122) umfasst.

8. Kniegelenkendoprothese nach Anspruch 6 oder 7, **dadurch gekennzeichnet, dass** das mindestens eine Sicherungselement (122) einen in Richtung der Verbindungsrichtung wirksamen zweiten Anschlag (126) für das mindestens eine erste Verbindungselement (110) in der Verbindungsstellung umfasst.

9. Kniegelenkendoprothese nach Anspruch 8, **dadurch gekennzeichnet, dass** der zweite Anschlag (126) eine in Richtung auf die erste Anschlagfläche (118) hin weisende zweite Anschlagfläche (128) umfasst.

10. Kniegelenkendoprothese nach einem der Ansprüche 6 bis 9, **dadurch gekennzeichnet, dass** die Sicherungseinrichtung (120) eine Rast- oder Schnappverbindungseinrichtung (130) umfasst zum definierten Festlegen des mindestens einen Sicherungselements (122) am mindestens einen ersten oder am mindestens einen zweiten Verbindungselement (110), dass die Rast- oder Schnappverbindungseinrichtung (130) mindestens ein erstes Rastglied (136) und mindestens ein mit diesem zusammenwirkendes zweites Rastglied (142) umfasst, welche in einer Raststellung kraft- und/oder formschlüssig in Eingriff stehen und in einer Entsicherungsstellung außer Eingriff stehen, und dass das mindestens eine erste Rastglied (136) am mindestens einen Sicherungselement (122) und dass das mindestens eine zweite Rastglied (142) am mindestens einen ersten oder am mindestens einen zweiten Verbindungselement (110) angeordnet oder ausgebildet ist, dass dem mindestens einen Sicherungselement mindestens ein vorspannendes Element (134) zugeordnet ist, um die Rast- oder Schnappverbindungseinrichtung (130) unter Vorspannung in der Raststellung zu halten.

11. Kniegelenkendoprothese nach Anspruch 10, **dadurch gekennzeichnet, dass**
a) das mindestens eine vorspannende Element (134) in Form eines Federelements (154), insbesondere in Form einer Blattfeder (156), ausgebildet ist
und/oder
b) das mindestens eine Sicherungselement (122) das mindestens eine vorspannende Element (134) umfasst
und/oder
c) das mindestens eine Sicherungselement (122) und das mindestens eine vorspannende Element (134) einstückig ausgebildet sind
und/oder
d) das mindestens eine vorspannende Element (134) das mindestens eine erste oder das mindestens eine zweite Rastglied (136, 142) trägt.

12. Kniegelenkendoprothese nach einem der voranstehenden Ansprüche, **dadurch gekennzeichnet, dass**
a) die Kniegelenkendoprothese eine zwischen der Tibiakomponente (14) und der Femurkomponente (18) angeordnete, an der Femurkomponente (18) oder der Tibiakomponente (14) gehaltene, insbesondere beweglich gelagerte, Meniskuskomponente (38) umfasst
und/oder
b) die Femurkomponente (18) und die Tibiakomponente (14) relativ zueinander um eine zweite Rotationsachse (164) rotierbar gelagert sind
und/oder
c) das zweite Gelenkelement (26) einen Gelenkzapfen (166) umfasst, welcher die erste und/oder die zweite Rotationsachse (46, 164) definiert und in eine Gelenkzapfenaufnahme (168) der Tibiakomponente (14) eingreift.

13. Kniegelenkendoprothese nach einem der voranstehenden Ansprüche, **gekennzeichnet durch** eine Luxationssicherungseinrichtung (174) zum Verhindern eines außer Eingriff Bringens des zweiten Gelenkelements (26) und der Tibiakomponente (14).

14. Kniegelenkendoprothese nach Anspruch 13, **dadurch gekennzeichnet, dass** die Luxationssicherungseinrichtung (174) mindestens ein erstes Verriegelungselement (206) und mindestens ein zweites Verriegelungselement (208) umfasst, die in einer Verriegelungsstellung in Eingriff stehen und in einer Freigabestellung außer Eingriff stehen, dass das mindestens eine erste Verriegelungselement (206) dem zweiten Gelenkelement (26) zugeordnet oder an diesem angeordnet oder ausgebildet ist und dass das mindestens eine zweite Verriegelungselement (208) der Tibiakomponente (14) zugeordnet oder an dieser angeordnet oder ausgebildet ist.

15. Kniegelenkendoprothese nach Anspruch 14, **dadurch gekennzeichnet, dass**
a) das mindestens eine erste Verriegelungselement (206) und das mindestens eine zweite Verriegelungselement (208) relativ zueinander bewegbar, insbesondere in einer Verriegelungsrichtung (218) quer zur zweiten Rotationsachse (164), angeordnet oder ausgebildet sind
und/oder
b) die Luxationssicherungseinrichtung (174) ein Blockierelement (220) umfasst zum Verhindern einer Bewegung des mindestens einen ersten Verriegelungselements (206) und des mindestens einen zweiten Verriegelungselements (208) relativ zueinander von der Verriegelungsstellung in die Freigabestellung
und/oder
c) das mindestens eine erste Verriegelungselement (206) und das mindestens eine zweite Verriegelungselement (208) in der Verriegelungsstellung relativ zueinander in einer Richtung parallel zur zweiten Rotationsachse (164) bewegbar sind
und/oder
d) das mindestens eine erste Verriegelungselement (206) in Form eines beweglich gelagerten Verriegelungskörpers (210) ausgebildet ist und dass das mindestens eine zweite Verriegelungselement (208) in Form einer Verriegelungsaufnahme (212) ausgebildet ist, in welcher der Verriegelungskörper (210) in der Verriegelungsstellung eingreift,
wobei insbesondere der Verriegelungskörper (210) in Form einer Kugel (214) ausgebildet ist, welche in einer quer zur zweiten Rotationsachse (164) verlaufenden Kugelbohrung (216) beweglich gehalten ist,
und/oder
e) das mindestens eine zweite Verriegelungselement (208) an einer Verriegelungshülse (176) angeordnet oder ausgebildet ist und dass die Verriegelungshülse (176) in der Gelenkzapfenaufnahme (168) kraft- und/oder formschlüssig gehalten ist,
wobei insbesondere die Verriegelungshülse (176) mit einem Rückhalteelement (196) in der Gelenkzapfenaufnahme (168) gehalten ist.

## Claims

1. Knee joint endoprosthesis (10) having a tibial component (14) and a femoral component (18) and a hinge joint (20) for coupling the tibial component (14) and the femoral component (18) so as to be pivotal about a rotational axis (22), which hinge joint comprises a first joint element (24) and a second joint element (26) coupled therewith so as to be rotatable about the rotational axis (22), wherein a connecting device (112) is provided having at least one first connecting element (108) and at least one second connecting element (110) for connecting the first joint element (24) to the femoral component (18), which connecting device (112) defines a connecting position, in which the at least one first connecting element (108) and the at least one second connecting element (110) are engaged in a non-positive- and/or positive-locking manner, and an assembly position, in which the first joint element (24) and the femoral component (18) are fully separated from each other, wherein the at least one first connecting element (108) is associated with the first joint element (24) or is arranged or formed thereon, and wherein the at least one second connecting element (110) is associated with the femoral component (18) or is arranged or formed thereon, wherein the connecting device (112) defines a connecting direction (114), in which the at least one first connecting element (108) and the at least one second connecting element (110) are moveable relative to each other for transferring the connecting device (112) from the assembly position into the connecting position, wherein the connecting direction (114) runs transversely, in particular perpendicularly, to the rotational axis (22), **characterized in that** the first joint element (24) is configured in the form of a hinge axle (72), and **in that** the second joint element (26) has a hinge axle receiver (74) that is passed through by the hinge axle (72).

2. Knee joint endoprosthesis in accordance with Claim 1, **characterized in that**
a) the second joint element (26) is held on the tibial component (14) or is coupleable therewith
and/or
b) the connecting device (112) is configured in such a way that it is transferrable from the assembly position into the connecting position after a mutually independent implantation of the femoral component (18) and the tibial component (14),
and/or
c) a longitudinal axis defined by the first joint element (24) defines the rotational axis (22)
and/or
d) the at least one first connecting element (108) is configured in the form of a connecting projection (94) and **in that** the at least one second connecting element (110) in the form of a connecting receiver (100), or vice versa,
wherein, in particular, the connecting receiver (100) extends in parallel to the connecting direction (114) and has an insertion opening (106) for the insertion of the connecting projection (94) in parallel to the connecting direction (114),
and/or
e) the knee joint endoprosthesis comprises two first and/or two second connecting elements (108, 110).

3. Knee joint endoprosthesis in accordance with any one of the preceding Claims, **characterized in that**
a) the hinge axle receiver (74) is configured in the form of a through-bore (76)
and/or
b) the hinge axle (72) and the hinge axle receiver (74) form a sliding bearing (80).

4. Knee joint endoprosthesis in accordance with any one of the preceding Claims, **characterized in that** the hinge axle (72) comprises a hinge axle core (82) and a hinge axle sleeve (84) arranged or mounted on the hinge axle core (82).

5. Knee joint endoprosthesis in accordance with Claim 4, **characterized in that**
a) the hinge axle sleeve (84) and the hinge axle receiver (74) define the sliding bearing (80)
and/or
b) the hinge axle core (82) in the connecting position is engaged with the at least one second connecting element (110) in a non-positive- and/or positive-locking manner
and/or
c) the hinge axle core (82) has a circular, oval, or an angular, in particular a rectangular or hexagonal, cross section
and/or
d) the hinge axle sleeve (84) has a hinge axle core receiver (90) for accommodating the hinge axle core, and **in that** the hinge axle core receiver has a free cross section corresponding to the hinge axle core (82)
and/or
e) the hinge axle core (82) projects out of the hinge axle sleeve (84) on both sides
and/or
f) the hinge axle core (82) projects out of the hinge axle receiver (74) on both sides
and/or
g) the hinge axle sleeve (84) projects out of the hinge axle receiver (74) on both sides.

6. Knee joint endoprosthesis in accordance with any one of the preceding Claims, **characterized by** a securing device (120) for securing the connecting device (112) in a securing position in a non-positive-and/or positive-locking manner when the connecting device (112) assumes the connecting position, and in that the securing device (120) comprises at least one securing element (122) for securing the at least one first and the at least one second connecting element (108, 110) in the securing position in a non-positive- and/or positive-locking manner when they assume the connecting position.

7. Knee joint endoprosthesis in accordance with Claim 6, **characterized in that**
a) the at least one securing element (122) is configured in the form of a closure element (124) for closing the insertion opening (106) of the connecting receiver (100) in the securing position
and/or
b) the securing device (120) comprises a latching or snapping connecting device (130) for fixing the at least one securing element (122) to the at least one first or to the at least one second connecting element (110) in a defined manner, **in that** the latching or snapping connecting device (130) comprises at least one first latching member (136) and at least one second latching member (142) cooperative therewith, which, in a latching position, are engaged in a non-positive- and/or positive-locking manner and, in a disengagement position, are disengaged, and **in that** the at least one first latching member (136) is arranged or formed on the at least one securing element (122), and **in that** the at least one second latching member (142) is arranged or formed on the at least one first or on the at least one second connecting element (110),
wherein, in particular,
b1) the at least one first and the at least one second latching member (136, 142) are engaged under pretension when in the latching position
and/or
b2) the knee joint endoprosthesis comprises two first and two second latching members (136, 142)
and/or
b3) the at least one first and the at least one second latching member (136, 142) are moveable relative to each other from the disengagement position into the latching position in a securing direction (158) and vice versa, and **in that** the securing direction (158) runs transversely to the connecting direction (114) and transversely to the rotational axis (22), in particular perpendicularly to the connecting direction (114) and perpendicularly to the rotational axis (22),
and/or
c) the knee joint endoprosthesis comprises two securing elements (122).

8. Knee joint endoprosthesis in accordance with Claim 6 or 7, **characterized in that** the at least one securing element (122) comprises a second stop (126), which is operative in the direction of the connecting direction, for the at least one first connecting element (110) in the connecting position.

9. Knee joint endoprosthesis in accordance with Claim 8, **characterized in that** the second stop (126) comprises a second stop face (128) pointing in the direction toward the first stop face (118).

10. Knee joint endoprosthesis in accordance with any one of Claims 6 to 9, **characterized in that** the securing device (120) comprises a latching or snapping connecting device (130) for fixing the at least one securing element (122) to the at least one first or to the at least one second connecting element (110) in a defined manner, **in that** the latching or snapping connecting device (130) comprises at least one first latching member (136) and at least one second latching member (142) cooperative therewith, which, in a latching position, are engaged in a non-positive- and/or positive-locking manner and, in a disengagement position, are disengaged, and **in that** the at least one first latching member (136) is arranged or formed on the at least one securing element (122), and **in that** the at least one second latching member (142) is arranged or formed on the at least one first or on the at least one second connecting element (110), **in that** the at least one pretensioning element (134) is associated with the at least one securing element in order to hold the latching or snapping connecting device (130) under pretension when in the latching position.

11. Knee joint endoprosthesis in accordance with Claim 10, **characterized in that**
a) the at least one pretensioning element (134) is configured in the form of a spring element (154), in particular in the form of a leaf spring (156),
and/or
b) the at least one securing element (122) comprises the at least one pretensioning element (134)
and/or
c) the at least one securing element (122) and the at least one pretensioning element (134) are integrally formed
and/or
d) the at least one pretensioning element (134) bears the at least one first or the at least one second latching member (136, 142).

12. Knee joint endoprosthesis in accordance with any one of the preceding Claims, **characterized in that**
a) the knee joint endoprosthesis comprises a meniscal component (38) which is arranged between the tibial component (14) and the femoral component (18), held, in particular moveably mounted, on the femoral component (18) or the tibial component (14)
and/or
b) the femoral component (18) and the tibial component (14) are mounted so as to be rotatable relative to each other about a second rotational axis (164)
and/or
c) the second joint element (26) comprises a joint pin (166) which defines the first and/or the second rotational axis (46, 164) and engages in a joint pin receiver (168) of the tibial component (14).

13. Knee joint endoprosthesis in accordance with any one of the preceding Claims, **characterized by** a luxation securing device (174) for preventing a disengagement of the second joint element (26) and the tibial component (14).

14. Knee joint endoprosthesis in accordance with Claim 13, **characterized in that** the luxation securing device (174) comprises at least one first locking element (206) and at least one second locking element (208) that, in a locking position, are engaged and, in a release position, are disengaged, **in that** the at least one first locking element (206) is associated with the second joint element (26) or is arranged or formed thereon, and **in that** the at least one second locking element (208) is associated with the tibial component (14) or is arranged or formed thereon.

15. Knee joint endoprosthesis in accordance with Claim 14, **characterized in that**
a) the at least one first locking element (206) and the at least one second locking element (208) are arranged or configured so as to be moveable relative to each other, in particular in a locking direction (218) transverse to the second rotational axis (164)
and/or
b) the luxation securing device (174) comprises a blocking element (220) for preventing a movement of the at least one first locking element (206) and the at least one second locking element (208) relative to each other from the locking position into the release positon
and/or
c) the at least one first locking element (206) and the at least one second locking element (208) in the locking position are moveable relative to each other in a direction parallel to the second rotational axis (164)
and/or
d) the at least one first locking element (206) is configured in the form of a moveably mounted locking body (210), and **in that** the at least one second locking element (208) is configured in the form of a locking receiver (212) in which the locking body (210) engages in the locking position (212),
wherein, in particular, the locking body (210) is configured in the form of a sphere (214) which is moveably held in a spherical bore (216) running transversely to the second rotational axis (164),
and/or
e) the at least one second locking element (208) is arranged or formed on a locking sleeve (176), and **in that** the locking sleeve (176) is held in the joint pin receiver (168) in a non-positive-and/or positive-locking manner
wherein, in particular, the locking sleeve (176) is held in the joint pin receiver (168) by a retaining element (196).

## Revendications

1. Endoprothèse d'articulation du genou (10) comprenant un composant de tibia (14) et un composant de fémur (18) et une articulation à charnière (20) pour assurer un couplage d'articulation pivotante du composant de tibia (14) et du composant de fémur (18) autour d'un axe de pivotement (22), ladite articulation à charnière comportant un premier élément d'articulation (24) et, couplé à celui-ci de manière rotative autour de l'axe de pivotement (22), un deuxième élément d'articulation (26), endoprothèse
dans laquelle il est prévu un dispositif de liaison (112) avec au moins un premier élément de liaison (108) et au moins un deuxième élément de liaison (110), pour assurer la liaison du premier élément d'articulation (24) avec le composant de fémur (18), ledit dispositif de liaison (112) définissant une position de liaison dans laquelle ledit au moins un premier élément de liaison (108) et ledit au moins un deuxième élément de liaison (110) sont en prise réciproque par adhérence et/ou complémentarité de formes, et une position de montage dans laquelle le premier élément d'articulation (24) et le composant de fémur (18) sont totalement séparés l'un de l'autre,
dans laquelle ledit au moins un premier élément de liaison (108) est associé au premier élément d'articulation (24) ou bien est agencé ou formé sur celui-ci, et dans laquelle ledit au moins un deuxième élément de liaison (110) est associé au composant de fémur (18) ou bien est agencé ou formé sur celui-ci, et dans laquelle le dispositif de liaison (112) définit une direction de liaison (114) dans laquelle ledit au moins un premier élément de liaison (108) et ledit au moins un deuxième élément de liaison (110) peuvent être déplacés l'un par rapport à l'autre pour le transfert du dispositif de liaison (112) de la position de montage à la position de liaison, la direction de liaison (114) s'étendant transversalement, notamment de manière perpendiculaire, à l'axe de pivotement (22), **caractérisée en ce que** le premier élément d'articulation (24) est réalisé sous la forme d'un axe de charnière (72), et **en ce que** le deuxième élément d'articulation (26) comporte un logement d'accueil d'axe de charnière (74), qui est traversé par l'axe de charnière (72).

2. Endoprothèse d'articulation du genou selon la revendication 1, **caractérisée en ce que**
a) le deuxième élément d'articulation (26) est maintenu sur le composant de tibia (14) ou peut être couplé à celui-ci,
et/ou
b) le dispositif de liaison (112) est réalisé de manière telle, qu'après une implantation indépendante du composant de fémur (18) et du composant de tibia (14), il puisse être transféré de la position de montage à la position de liaison, et/ou
c) un axe longitudinal défini par le premier élément d'articulation (24), définit l'axe de pivotement (22),
et/ou
d) ledit au moins un premier élément de liaison (108) est réalisé sous la forme d'une protubérance de liaison (94) et ledit au moins un deuxième élément de liaison (110) sous la forme d'un logement d'accueil de liaison (100), ou inversement,
le logement d'accueil de liaison (100) s'étendant notamment parallèlement à la direction de liaison (114), et présentant une ouverture d'introduction (106) pour l'introduction de la protubérance de liaison (94) parallèlement à la direction de liaison (114),
et/ou
e) l'endoprothèse d'articulation de genou comprend deux premiers et/ou deux deuxièmes éléments de liaison (108, 110).

3. Endoprothèse d'articulation du genou selon l'une des revendications précédentes, **caractérisée en ce que**
a) le logement d'accueil d'axe de charnière (74) est réalisé sous la forme d'un alésage traversant (76), et/ou
b) l'axe de charnière (72) et le logement d'accueil d'axe de charnière (74) forment un palier lisse (80) .

4. Endoprothèse d'articulation du genou selon l'une des revendications précédentes, **caractérisée en ce que** l'axe de charnière (72) comprend une âme d'axe de charnière (82) et un coussinet d'axe de charnière (84) agencé ou monté sur l'âme d'axe de charnière (82).

5. Endoprothèse d'articulation du genou selon la revendication 4, **caractérisée en ce que**
a) le coussinet d'axe de charnière (84) et le logement d'accueil d'axe de charnière (74) définissent le palier lisse (80),
et/ou
b) l'âme d'axe de charnière (82) est, dans la position de liaison, en prise par adhérence et/ou par complémentarité de formes avec ledit au moins un deuxième élément de liaison (110),
et/ou
c) l'âme d'axe de charnière (82) présente une section transversale circulaire, ovale ou polygonale, notamment carrée ou hexagonale,
et/ou
d) le coussinet d'axe de charnière (84) présente un logement d'accueil d'âme d'axe de charnière (90) destiné à accueillir l'âme d'axe de charnière, et **en ce que** le logement d'accueil d'âme d'axe de charnière présente une section transversale libre correspondant à celle de l'âme d'axe de charnière (82),
et/ou
e) l'âme d'axe de charnière (82) fait saillie des deux côtés du coussinet d'axe de charnière (84),
et/ou
f) l'âme d'axe de charnière (82) fait saillie des deux côtés du logement d'accueil d'axe de charnière (74), et/ou
g) le coussinet d'axe de charnière (84) fait saillie des deux côtés du logement d'accueil d'axe de charnière (74).

6. Endoprothèse d'articulation du genou selon l'une des revendications précédentes, **caractérisée par** un dispositif de verrouillage de sécurisation (120) pour réaliser le verrouillage sécurisé par adhérence et/ou complémentarité de formes du dispositif de liaison (112) dans une position de verrouillage de sécurisation lorsque le dispositif de liaison (112) prend la position de liaison, et en ce que le dispositif de verrouillage de sécurisation (120) comprend au moins un élément de verrouillage de sécurisation (122) pour assurer l'arrêt sécurisé par adhérence et/ou par complémentarité de formes dudit au moins un premier et dudit au moins un deuxième élément de liaison (108, 110) dans la position de verrouillage sécurisée lorsque ceux-ci prennent la position de liaison.

7. Endoprothèse d'articulation du genou selon la revendication 6, **caractérisée en ce que**
a) ledit au moins un élément de verrouillage de sécurisation (122) est réalisé sous la forme d'un élément de fermeture (124) pour fermer l'ouverture d'introduction (106) du logement d'accueil de liaison (100) dans la position de verrouillage de sécurisation,
et/ou
b) le dispositif de verrouillage de sécurisation (120) comprend un système de liaison d'encliquetage ou d'enclenchement (130) pour la fixation définie dudit au moins un élément de verrouillage de sécurisation (122) sur ledit au moins un premier ou ledit au moins un deuxième élément de liaison (110), **en ce que** le système de liaison d'encliquetage ou d'enclenchement (130) comprend au moins un premier organe d'encliquetage (136) et au moins un deuxième organe d'encliquetage (142) interagissant avec le précédent, ces organes d'encliquetage étant en prise réciproque par adhérence et/ou par complémentarité de formes dans une position encliquetée, et étant hors de prise dans une position de déverrouillage, et **en ce que** ledit au moins un premier organe d'encliquetage (136) est agencé ou réalisé sur ledit au moins un élément de verrouillage de sécurisation (122) et ledit au moins un deuxième organe d'encliquetage (142) sur ledit au moins un premier ou ledit au moins un deuxième élément de liaison (110),
ensemble pour lequel notamment
b1) ledit au moins un premier et ledit au moins un deuxième organe d'encliquetage (136, 142) sont en prise réciproque sous précontrainte, dans la position encliquetée,
et/ou
b2) l'endoprothèse d'articulation du genou comprend deux premiers et deux deuxièmes organes d'encliquetage (136, 142),
et/ou
b3) ledit au moins un premier et ledit au moins un deuxième organe d'encliquetage (136, 142) peuvent être déplacés relativement l'un par rapport à l'autre de la position de déverrouillage à la position d'encliquetage dans une direction de verrouillage de sécurisation (158), et inversement, et **en ce que** la direction de verrouillage de sécurisation (158) s'étend transversalement à la direction de liaison (114) et transversalement à l'axe de pivotement (22), notamment perpendiculairement à la direction de liaison (114) et perpendiculairement à l'axe de pivotement (22),
et/ou
c) l'endoprothèse d'articulation du genou comprend deux éléments de verrouillage de sécurité (122).

8. Endoprothèse d'articulation du genou selon la revendication 6 ou la revendication 7, **caractérisée en ce que** ledit au moins un élément de verrouillage de sécurisation (122) comporte une deuxième butée (126) agissant dans le sens de la direction de liaison pour ledit au moins un premier élément de liaison (110) dans la position de liaison.

9. Endoprothèse d'articulation du genou selon la revendication 8, **caractérisée en ce que** la deuxième butée (126) comporte une deuxième surface de butée (128) dirigée en direction de la première surface de butée (118) .

10. Endoprothèse d'articulation du genou selon l'une des revendications 6 à 9, **caractérisée en ce que** le dispositif de verrouillage de sécurisation (120) comprend un système de liaison d'encliquetage ou d'enclenchement (130) pour la fixation définie dudit au moins un élément de verrouillage de sécurisation (122) sur ledit au moins un premier ou ledit au moins un deuxième élément de liaison (110), **en ce que** le système de liaison d'encliquetage ou d'enclenchement (130) comprend au moins un premier organe d'encliquetage (136) et au moins un deuxième organe d'encliquetage (142) interagissant avec le précédent, ces organes d'encliquetage étant en prise réciproque par adhérence et/ou par complémentarité de formes dans une position encliquetée, et étant hors de prise dans une position de déverrouillage, et **en ce que** ledit au moins un organe d'encliquetage (136) est agencé ou réalisé sur ledit au moins un élément de verrouillage de sécurisation (122) et ledit au moins un deuxième organe d'encliquetage (142) sur ledit au moins un premier ou ledit au moins un deuxième élément de liaison (110), et **en ce qu'**au dit au moins un élément de verrouillage de sécurisation, est associé au moins un élément de précontrainte (134) pour maintenir le système de liaison d'encliquetage ou d'enclenchement (130) sous précontrainte, dans la position encliquetée.

11. Endoprothèse d'articulation du genou selon la revendication 10, **caractérisée en ce que**
a) ledit au moins un élément de précontrainte (134) est réalisé sous la forme d'un élément de ressort (154), notamment sous la forme d'un ressort à lame (156), et/ou
b) ledit au moins un élément de verrouillage de sécurisation (122) comprend ledit au moins un élément de précontrainte (134),
et/ou
c) ledit au moins un élément de verrouillage de sécurisation (122) et ledit au moins un élément de précontrainte (134) sont réalisés d'un seul tenant, et/ou
d) ledit au moins un élément de précontrainte (134) porte ledit au moins un premier ou ledit au moins un deuxième organe d'encliquetage (136, 142).

12. Endoprothèse d'articulation du genou selon l'une des revendications précédentes, **caractérisée en ce que**
a) l'endoprothèse d'articulation du genou comprend un composant de ménisque (38), qui est agencé entre le composant de tibia (14) et le composant de fémur (18), et qui est maintenu, notamment monté de manière mobile, sur le composant de fémur (18) ou sur le composant de tibia (14),
et/ou
b) le composant de fémur (18) et le composant de tibia (14) sont montés rotatifs l'un par rapport à l'autre autour d'un deuxième axe de rotation (164),
et/ou
c) le deuxième élément d'articulation (26) comporte un tourillon d'articulation (166), qui définit le premier et/ou le deuxième axe de rotation (46, 164) et vient s'engager dans un logement d'accueil de tourillon d'articulation (168) du composant de tibia (14) .

13. Endoprothèse d'articulation du genou selon l'une des revendications précédentes, **caractérisée par** un système de sécurité anti-luxation (174) pour empêcher une désolidarisation du deuxième élément d'articulation (26) et du composant de tibia (14).

14. Endoprothèse d'articulation du genou selon la revendication 13, **caractérisée en ce que** le système de sécurité anti-luxation (174) comprend au moins un premier élément de verrouillage (206) et au moins un deuxième élément de verrouillage (208), qui sont en prise réciproque dans une position de verrouillage, et sont hors de prise dans une position de dégagement, **en ce que** ledit au moins un premier élément de verrouillage (206) est associé au deuxième élément d'articulation (26) ou agencé ou formé sur celui-ci, et **en ce que** ledit au moins un deuxième élément de verrouillage (208) est associé au composant de tibia (14) ou agencé ou formé sur celui-ci.

15. Endoprothèse d'articulation du genou selon la revendication 14, **caractérisée en ce que**
a) ledit au moins un premier élément de verrouillage (206) et ledit au moins un deuxième élément de verrouillage (208) sont agencés ou réalisés de manière à pouvoir être déplacés l'un par rapport à l'autre, notamment dans une direction de verrouillage (218), transversalement au deuxième axe de rotation (164),
et/ou
b) le système de sécurité anti-luxation (174) comporte un élément de blocage (220) pour empêcher un mouvement relatif dudit au moins un premier élément de verrouillage (206) et dudit au moins un deuxième élément de verrouillage (208) l'un par rapport à l'autre, de la position de verrouillage à la position de dégagement,
et/ou
c) ledit au moins un premier élément de verrouillage (206) et ledit au moins un deuxième élément de verrouillage (208) peuvent, dans la position de verrouillage, être déplacés relativement l'un par rapport à l'autre dans une direction parallèle au deuxième axe de rotation (164),
et/ou
d) ledit au moins un premier élément de verrouillage (206) est réalisé sous la forme d'un corps de verrouillage (210) monté mobile, et **en ce que** ledit au moins un deuxième élément de verrouillage (208) est réalisé sous la forme d'un logement d'accueil de verrouillage (212), dans lequel s'engage le corps de verrouillage (210) dans la position de verrouillage, le corps de verrouillage (210) étant notamment réalisé sous la forme d'une bille (214), qui est maintenue mobile dans un alésage de bille (216) s'étendant transversalement au deuxième axe de rotation (164),
et/ou
e) ledit au moins un deuxième élément de verrouillage (208) est agencé ou formé sur une douille de verrouillage (176), et **en ce que** la douille de verrouillage (176) est maintenue par adhérence et/ou par complémentarité de formes dans le logement d'accueil de tourillon d'articulation (168),
la douille de verrouillage (176) étant notamment maintenue dans le logement d'accueil de tourillon d'articulation (168) au moyen d'un élément de retenue (196).
